# EUROPEAN PATENT APPLICATION

(11) **EP 3 438 090 A1**
(43) Date of publication of application: **06.02.2019**
(21) Application number: 17775418.1
(22) Date of filing: 30.03.2017
(51) Int. Cl.: C07C 259/06, C07C 27/02, C07C 33/26, C07D 317/12

(54) **NOVEL METHOD FOR PRODUCING HYDROXAMIC ACID DERIVATIVE, AND INTERMEDIATE THEREOF**

(30) Priority: 31.03.2016 JP 2016070503
(71) Applicant: Toyama Chemical Co., Ltd., Shinjuku-ku Tokyo 160-0023 (JP)
(72) Inventor: NAGATO, Yusuke, Toyama-shi Toyama 930-8508 (JP); BABA, Yasutaka, Toyama-shi Toyama 930-8508 (JP)
(74) Representative: Hoffmann Eitle
(86) International application number: PCT/JP2017/013289
(87) International publication number: WO 2017/170884

(57) **Abstract**

A novel method for production of hydroxamic acid derivative using a compound represented by the formula [12] as an intermediate, and intermediate therefor. 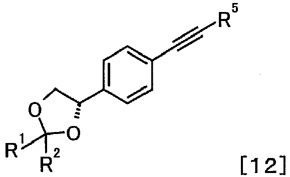 wherein R¹ represents a methyl group or the like; R² represents a methyl group or the like; and R⁵ represents a hydrogen atom or the like.

## Description

### Technical Field

The present invention relates to a method for producing (2S)-2-((4-((4-((1S)-1,2-dihydroxyethyl)phenyl)ethynyl)benzoyl)(methyl)amino)-N-hydroxy-N',2-dimethylmalonamide having excellent inhibitory activity against uridyldiphospho-3-O-acyl-N-acetylglucosamine deacetylase (LpxC), and an intermediate therefor.

### Background Art

LpxC is an enzyme that catalyzes the synthesis of lipid A. The lipid A is a component essential for outer membrane formation and is essential for, for example, the survival of gram-negative bacterial. Thus, a drug inhibiting the activity of LpxC is strongly expected to be able to serve as an antibacterial agent effective for gram-negative bacteria including *Pseudomonas aeruginosa.*

For example, (2S)-2-((4-((4-((1S)-1,2-dihydroxyethyl)phenyl)ethynyl)benzoyl)(methyl)amino)-N-hydroxy-N',2-dimethylmalonamide (formula [A]; hereinafter, also referred to as compound A) having excellent LpxC inhibitory activity, and a method for producing this compound are known (Patent Literature 1).

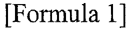

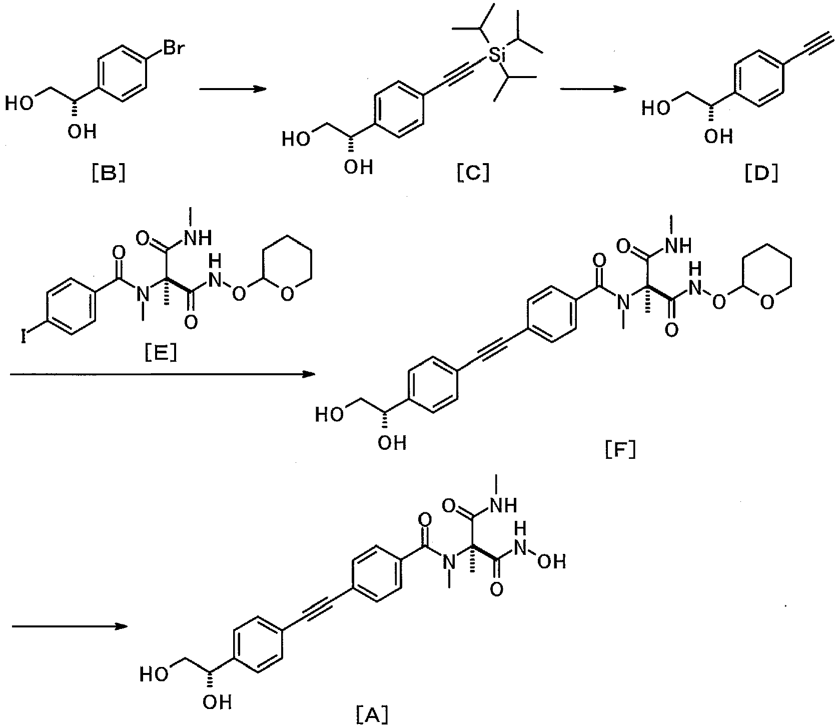

Meanwhile, a method for producing an optically active diol compound is known (Non Patent Literature 1).

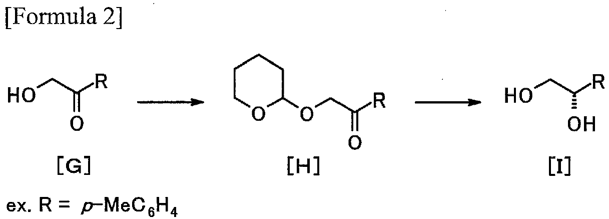

### Prior Art Literatures

### Patent Literature

Patent Literature 1: International Publication No. WO 2014/142298 pamphlet

### Non Patent Literature

Non Patent Literature 1: Tetrahedron: Asymmetry 10, (1999), p. 1843-1846

### Summary of Invention

### Object to be Solved by the Invention

Non Patent Literature 1 describes a method for producing a compound represented by the formula [I] by protecting the hydroxyl group in a compound represented by the formula [G], followed by asymmetric reduction reaction. However, this production method has the following disadvantages:
(1) The compound represented by the formula [G] is unstable.
(2) Therefore, the compound represented by the formula [G] is difficult to produce.
(3) The unstable compound represented by formula [G] must be used for producing a compound represented by the formula [H].

Patent Literature 1 describes a method for producing compound A. However, this production method has the following disadvantages:
(1) Column chromatography is necessary for the purification of a compound represented by formula [D].
(2) Column chromatography is necessary for the aftertreatment of the production of a compound represented by formula [F] from the compound represented by formula [D].
(3) Column chromatography is necessary for the purification of compound A.

Compound A has asymmetric carbon in the molecule. Therefore, in the case of using compound A as a bulk pharmaceutical, it is strongly desired to provide a compound having high optical purity.

An object of the present invention is to provide an industrial method for producing compound A having high optical purity and excellent LpxC inhibitory activity, and an intermediate therefor.

### Means for Solving the Object

Under these circumstances, the present inventors have conducted diligent studies and consequently found an industrial method for producing compound A having high optical purity. This production method is performed by convenient operation and is safe to human bodies. The present inventors have further found a useful intermediate for use in this production. On the basis of these findings, the present invention has been completed.

The present invention provides the following subject matters.
[1] A method for producing a compound represented by formula [2], comprising a step of:
   deprotecting a compound represented by the formula [1]: 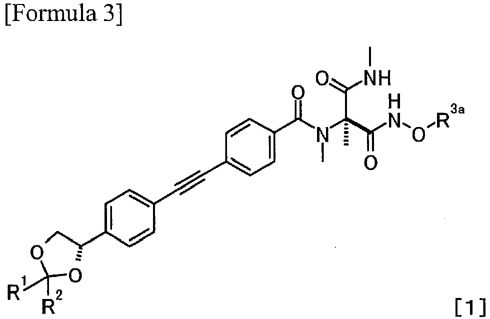
   wherein R¹ represents a C₁₋₃ alkyl group; R² represents a C₁₋₃ alkyl group; and R^{3a} represents a hydroxyl-protecting group
   to produce the compound represented by formula [2]: 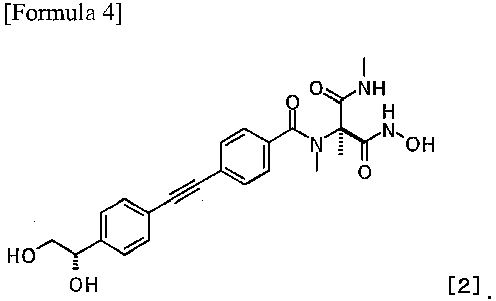
[2] The production method according to [1], wherein the step of deprotecting the compound represented by the formula [1] is a step comprising steps of:
   (1) deprotecting the hydroxyl-protecting group in the compound represented by the formula [1] to obtain a compound represented by the formula [3]: 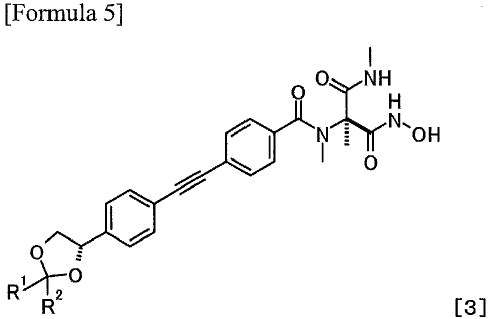 wherein R¹ and R² are as defined above; and
   (2) deprotecting the compound represented by the formula [3] to obtain the compound represented by formula [2]: 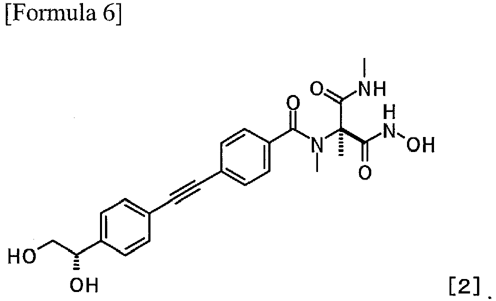
[3] The production method according to [1] or [2], wherein R¹ is a methyl group; and R² is a methyl group.
[4] The production method according to any one of [1] to [3], wherein R^{3a} is a tetrahydropyranyl group.
[5] A method for producing a compound represented by formula [2], comprising steps of:
   reacting a compound represented by the formula [4]: 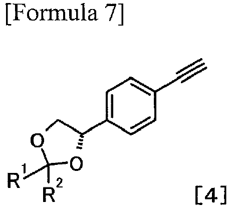
   wherein R¹ and R² are as defined above;
   with a compound represented by the formula [5]: 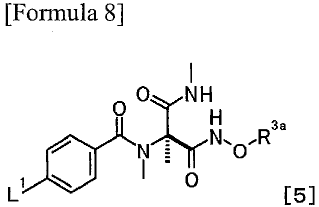
   wherein L¹ represents a leaving group and R^{3a} is as defined above;
   to obtain a compound represented by the formula [1]: 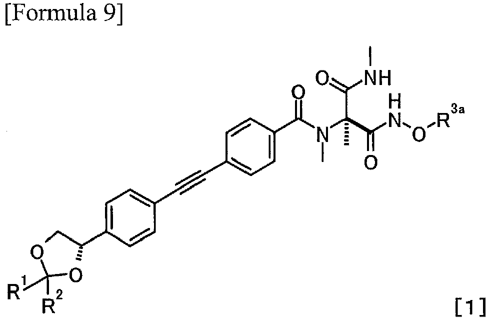
   wherein R¹, R², and R^{3a} are as defined above; and
   deprotecting the obtained compound represented by the formula [1] to produce the compound represented by formula [2]: 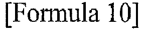 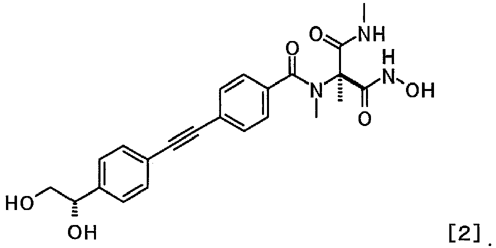
[6] The production method according to [5], wherein the step of deprotecting the compound represented by the formula [1] is a step comprising steps of:
   (1) deprotecting the hydroxyl-protecting group in the compound represented by the formula [1] to obtain a compound represented by the formula [3]: 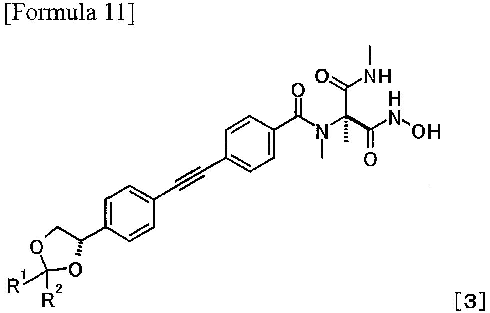 wherein R¹ and R² are as defined above; and
   (2) deprotecting the compound represented by the formula [3] to obtain the compound represented by formula [2]: 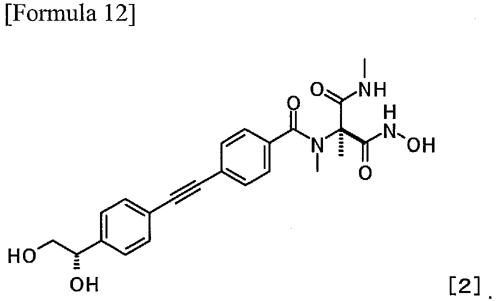
[7] The production method according to [5] or [6], wherein R¹ is a methyl group; and R² is a methyl group.
[8] The production method according to any one of [5] to [7], wherein R^{3a} is a tetrahydropyranyl group; and L¹ is an iodine atom.
[9] A method for producing a compound represented by formula [2], comprising steps of:
   reducing a compound represented by the formula [6]: 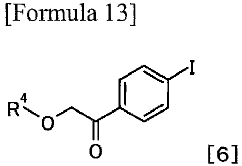
   wherein R⁴ represents an optionally substituted acyl group
   to obtain a compound represented by the formula [7]: 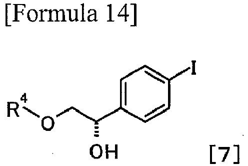
   wherein R⁴ is as defined above;
   deprotecting the obtained compound represented by the formula [7] to obtain a compound represented by formula [8]: 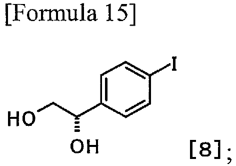
   reacting the obtained compound represented by formula [8] with a compound represented by the formula [9]: 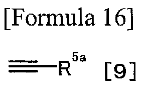
   wherein R^{5a} represents a silyl group
   to obtain a compound represented by the formula [10]: 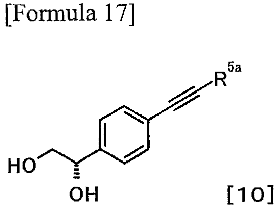
   wherein R^{5a} is as defined above;
   deprotecting the obtained compound represented by the formula [10] to obtain a compound represented by formula [11]: 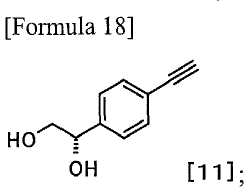
   protecting the diol group in the obtained compound represented by formula [11] to obtain a compound represented by the formula [4]: 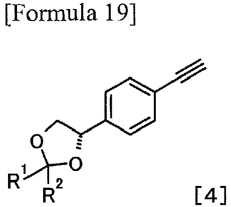
   wherein R¹ and R² are as defined above;
   reacting the obtained compound represented by the formula [4] with a compound represented by the formula [5]: 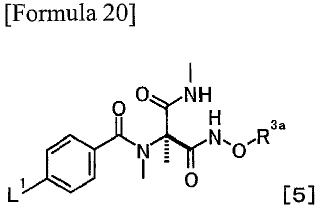
   wherein R^{3a} and L¹ are as defined above
   to obtain a compound represented by the formula [1]: 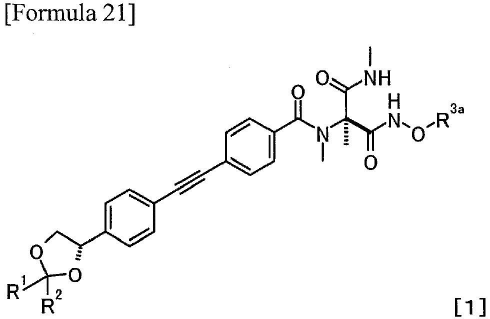
   wherein R¹, R², and R^{3a} are as defined above; and
   deprotecting the obtained compound represented by the formula [1] to produce the compound represented by formula [2]: 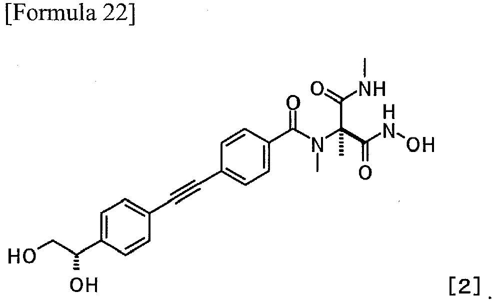
[10] The production method according to [9], wherein the step of deprotecting the compound represented by the formula [1] is a step comprising steps of:
   (1) deprotecting the hydroxyl-protecting group in the compound represented by the formula [1] to obtain a compound represented by the formula [3]: 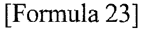 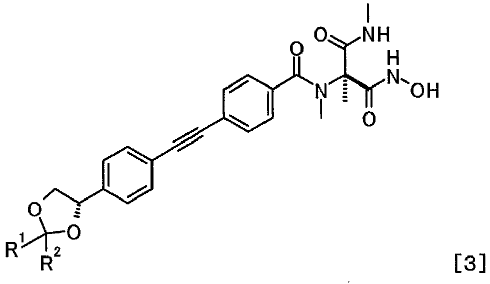 wherein R¹ and R² are as defined above; and
   (2) deprotecting the compound represented by the formula [3] to obtain the compound represented by formula [2]: 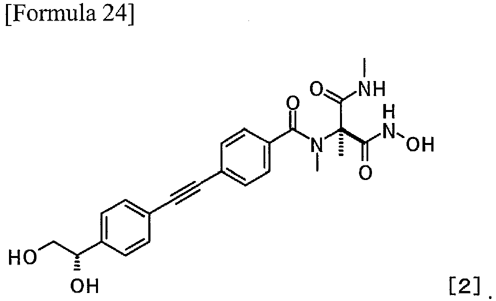
[11] The production method according to [9] or [10], wherein R⁴ is an acetyl group; and R^{5a} is a trimethylsilyl group.
[12] The production method according to any one of [9] to [11], wherein R¹ is a methyl group; and R² is a methyl group.
[13] The production method according to any one of [9] to [12], wherein R^{3a} is a tetrahydropyranyl group; and L¹ is an iodine atom.
[14] A method for producing a compound represented by formula [8], comprising steps of:
   reducing a compound represented by the formula [6]: 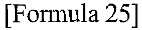 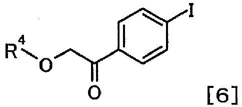
   wherein R^{4a} is as defined above
   to obtain a compound represented by the formula [7]: 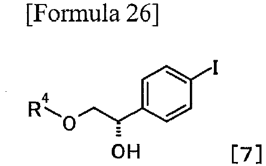
   wherein R⁴ is as defined above; and
   deprotecting the obtained compound represented by the formula [7] to produce the compound represented by formula [8]: 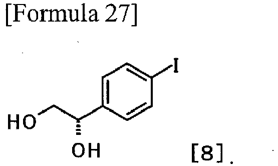
[15] The production method according to [14], wherein R⁴ is an acetyl group.
[16] A compound represented by the formula [12]: 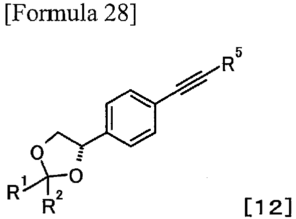 wherein R⁵ represents a hydrogen atom, a group represented by the formula [13]: 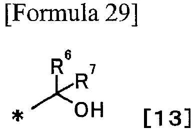 wherein R⁶ represents a C₁₋₃ alkyl group; R⁷ represents a C₁₋₃ alkyl group; or R⁶ and R⁷ together represent a C₃₋₆ alkylene group; and * represents a binding position, or a group represented by the formula [14]: 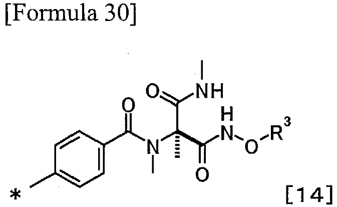 wherein R³ represents a hydrogen atom or a hydroxyl-protecting group; and * represents a binding position; and R¹ and R² are as defined above.
[17] The compound according to [16], wherein R¹ is a methyl group; R² is a methyl group; and R⁵ is a hydrogen atom.
[18] The compound according to [16], wherein R¹ is a methyl group; R² is a methyl group; and R⁵ is a group represented by the formula [14]: 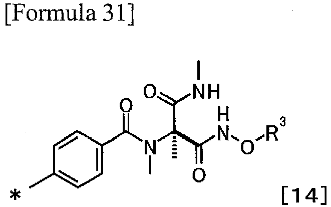 wherein R³ and * are as defined above.
[19] The compound according to [18], wherein R³ is a hydrogen atom or a tetrahydropyranyl group.

The present invention further provides the following:
[A] A method for producing a compound represented by the formula [4], comprising steps of:
   reacting a compound represented by formula [8]: 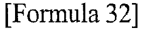 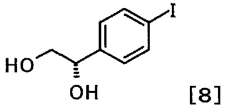
   with a compound represented by the formula [9]: 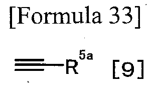
   wherein R^{5a} is as defined above
   to obtain a compound represented by the formula [10]: 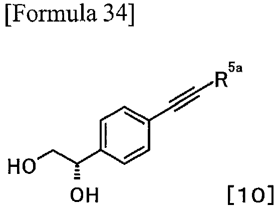
   wherein R^{5a} is as defined above;
   deprotecting the obtained compound represented by the formula [10] to obtain a compound represented by formula [11]: 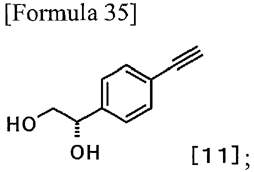 and
   protecting the diol group in the obtained compound represented by formula [11] to produce the compound represented by the formula [4]: 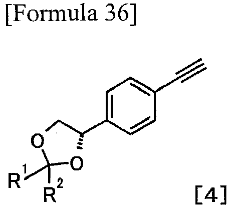
   wherein R¹ and R² are as defined above.
[B] The production method according to [A], wherein R^{5a} is a trimethylsilyl group.
[C] The production method according to [A] or [B], wherein R¹ is a methyl group; and R² is a methyl group.
[D] A method for producing a compound represented by the formula [4], comprising steps of:
   protecting the diol group in a compound represented by formula [8]: 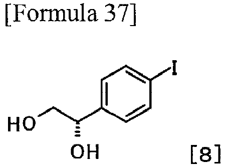
   to obtain a compound represented by the formula [15]: 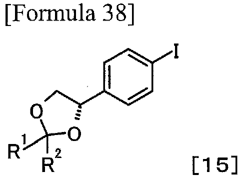
   wherein R¹ and R² are as defined above;
   reacting the obtained compound represented by the formula [15] with a compound represented by the formula [16]: 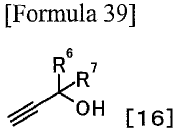
   wherein R⁶ represents a C₁₋₃ alkyl group; and R⁷ represents a C₁₋₃ alkyl group; or R⁶ and R⁷ together represent a C₃₋₆ alkylene group
   to obtain a compound represented by the formula [17]: 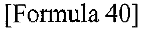 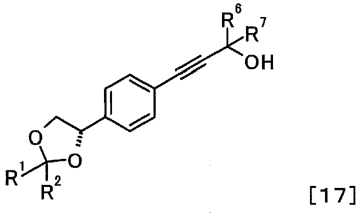
   wherein R¹, R², R⁶, and R⁷ are as defined above; and
   deprotecting the obtained compound represented by the formula [17] to produce the compound represented by the formula [4]: 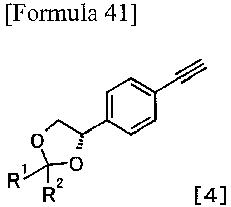
   wherein R¹ and R² are as defined above.
[E] The production method according to [D], wherein R⁶ is a methyl group; and R⁷ is a methyl group.
[F] The production method according to [D] or [E], wherein R¹ is a methyl group; and R² is a methyl group.

### Advantageous Effects of Invention

Features of the production method of the present invention are that: an intermediate is easily handleable; the production method is performed by convenient operation and is safe to human bodies; the resulting compound has high optical purity; etc. Therefore, the production method of the present invention is useful as an industrial method for producing compound A having high optical purity and excellent LpxC inhibitory activity.

The compound of the present invention is useful as an intermediate for use in the industrial production of compound A having high optical purity and excellent LpxC inhibitory activity.

### Brief Description of Drawings

[Figure 1] Figure 1 is a view showing an example of attenuated total reflection infrared spectroscopy (ATR method) performed on the type II crystal of the hydrate of Compound A (Example 16).
[Figure 2] Figure 2 is a view showing an example of the powder X-ray diffraction pattern of the type II crystal of the hydrate of Compound A (Example 16).
[Figure 3] Figure 3 is a view showing an example of attenuated total reflection infrared spectroscopy (ATR method) performed on the type III crystal of Compound A (Example 17).
[Figure 4] Figure 4 is a view showing an example of the powder X-ray diffraction pattern of the type III crystal of Compound A (Example 17).

### Embodiments for Carrying out the Invention

Hereinafter, the present invention will be described in detail.

In the present invention, % is % by mass unless otherwise specified.

In the present invention, each term has the following meaning unless otherwise specified.

A halogen atom means a fluorine atom, a chlorine atom, a bromine atom, or an iodine atom.

A C₁₋₃ alkyl group means a methyl, ethyl, propyl, or isopropyl group.

A C₁₋₆ alkyl group means a linear or branched C₁₋₆ alkyl group such as a methyl, ethyl, propyl, isopropyl, butyl, sec-butyl, isobutyl, tert-butyl, pentyl, isopentyl, or hexyl group.

An ar-C₁₋₆ alkyl group means an ar-C₁₋₆ alkyl group such as a benzyl, diphenylmethyl, trityl, phenethyl, 2-phenylpropyl, 3-phenylpropyl, or naphthylmethyl group.

A C₃₋₆ alkylene group means a linear or branched C₃₋₆ alkylene group such as methylethylene, trimethylene, ethylethylene, tetramethylene, pentamethylene, or hexamethylene.

A C₁₋₆ alkoxy group means a linear, branched, or cyclic C₁₋₆ alkyloxy group such as a methoxy, ethoxy, propoxy, isopropoxy, cyclopropoxy, butoxy, isobutoxy, sec-butoxy, tert-butoxy, cyclobutoxy, pentyloxy, or hexyloxy group.

A C₁₋₆ alkoxy-C₁₋₆ alkyl group means a C₁₋₆ alkyloxy-C₁₋₆ alkyl group such as a methoxymethyl or 1-ethoxyethyl group.

A C₂₋₆ alkanoyl group means a linear or branched C₂₋₆ alkanoyl group such as an acetyl, propionyl, valeryl, isovaleryl, or pivaloyl group.

An aroyl group means a benzoyl or naphthoyl group.

An acyl group means a formyl group, a C₂₋₆ alkanoyl group, or an aroyl group.

A C₁₋₆ alkoxycarbonyl group means a linear or branched C₁₋₆ alkyloxycarbonyl group such as a methoxycarbonyl, ethoxycarbonyl, isopropoxycarbonyl, tert-butoxycarbonyl, or 1,1-dimethylpropoxycarbonyl group.

An ar-C₁₋₆ alkoxycarbonyl group means an ar-C₁₋₆ alkyloxycarbonyl group such as a benzyloxycarbonyl or phenethyloxycarbonyl group.

A C₁₋₆ alkylsulfonyl group means a C₁₋₆ alkylsulfonyl group such as a methylsulfonyl, ethylsulfonyl, or propylsulfonyl group.

An arylsulfonyl group means a benzenesulfonyl, p-toluenesulfonyl, or naphthalenesulfonyl group.

A C₁₋₆ alkylsulfonyloxy group means a C₁₋₆ alkylsulfonyloxy group such as a methylsulfonyloxy, ethylsulfonyloxy, and propylsulfonyloxy group.

An arylsulfonyloxy group means a benzenesulfonyloxy, p-toluenesulfonyloxy, or naphthalenesulfonyloxy group.

A silyl group means a trimethylsilyl, triethylsilyl, tri(isopropyl)silyl, tributylsilyl, or dimethyl(tert-butyl)silyl group.

A hydroxyl-protecting group includes every group that may be usually used as a protecting group for a hydroxyl group. Examples thereof include groups described in Greene's Protective Groups in Organic Synthesis, 5th ed., p. 17-471, 2014, John Wiley & Sons, INC. Specific examples thereof include a C₁₋₆ alkyl group, an ar-C₁₋₆ alkyl group, a C₁₋₆ alkoxy-C₁₋₆ alkyl group, an acyl group, a C₁₋₆ alkoxycarbonyl group, an ar-C₁₋₆ alkoxycarbonyl group, a C₁₋₆ alkylsulfonyl group, an arylsulfonyl group, a silyl group, a tetrahydrofuranyl group, or a tetrahydropyranyl group. These groups may each be substituted by one or two or more groups selected from substituent group A.

Examples of a leaving group include a halogen atom, a C₁₋₆ alkylsulfonyloxy group, or an arylsulfonyloxy group. The C₁₋₆ alkylsulfonyloxy group and the arylsulfonyloxy group may each be substituted by one or two or more groups selected from substituent group A.

Substituent group A: a halogen atom, a nitro group, a C₁₋₆ alkyl group, and a C₁₋₆ alkoxy group.

Aliphatic hydrocarbons mean pentane, hexane, heptane, cyclohexane, methylcyclohexane, or ethylcyclohexane.

Halogenated hydrocarbons mean dichloromethane, chloroform, or dichloroethane.

Ethers mean diethyl ether, diisopropyl ether, methyl (tert-butyl) ether, tetrahydrofuran, 2-methyltetrahydrofuran, 1,4-dioxane, anisole, ethylene glycol dimethyl ether, diethylene glycol dimethyl ether, and diethylene glycol diethyl ether.

Alcohols mean methanol, ethanol, propanol, 2-propanol, butanol, 2-methyl-2-propanol, ethylene glycol, propylene glycol, or diethylene glycol.

Ketones mean acetone, 2-butanone, or 4-methyl-2-pentanone.

Esters mean methyl acetate, ethyl acetate, propyl acetate, isopropyl acetate, or butyl acetate.

Amides mean N,N-dimethylformamide, N,N-dimethylacetamide, or N-methylpyrrolidone.

Nitriles mean acetonitrile or propionitrile.

Sulfoxides mean dimethyl sulfoxide or sulfolane.

Aromatic hydrocarbons mean benzene, toluene, or xylene.

An inorganic base means sodium hydroxide, potassium hydroxide, sodium bicarbonate, sodium carbonate, sodium hydride, potassium carbonate, tripotassium phosphate, potassium acetate, cesium fluoride, or cesium carbonate.

An organic base means sodium methoxide, sodium ethoxide, sodium tert-butoxide, potassium tert-butoxide, triethylamine, N,N-diisopropylethylamine, 1,8-diazabicyclo(5.4.0)undec-7-ene (DBU), pyridine, N,N-dimethyl-4-aminopyridine, or 4-methylmorpholine.

An inorganic acid means hydrochloric acid, hydrobromic acid, nitric acid, or sulfuric acid.

An organic acid means an organic carboxylic acid (e.g., formic acid, acetic acid, citric acid, oxalic acid, fumaric acid, maleic acid, succinic acid, malic acid, tartaric acid, aspartic acid, trichloroacetic acid, and trifluoroacetic acid) or an organic sulfonic acid (e.g., methanesulfonic acid, benzenesulfonic acid, p-toluenesulfonic acid, mesitylenesulfonic acid, and naphthalenesulfonic acid).

Examples of salts of the compounds represented by the formulas [2] and [3] include usually known salts of acidic groups such as a hydroxyl group.

Examples of the salts of acidic groups include: salts with alkali metals such as sodium and potassium; salts with alkaline earth metals such as calcium and magnesium; ammonium salts; and salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

Among the salts described above, examples of a preferred salt include pharmacologically acceptable salts.

When the compound used in the present invention has an anhydride, a solvate, a hydrate, and crystals in various forms, the present invention encompasses all of them.

In general, the diffraction angle (2θ) in the powder X-ray diffraction may cause an error in the range of ±0.2°. Accordingly, the term "diffraction angle (2θ) X°" is used in the present description to mean "diffraction angle (2θ) ((X - 0.2) to (X + 0.2))°", unless otherwise specified.

Besides, it has been known that the relative intensity in the powder X-ray diffraction fluctuates depending on crystal orientation, the size of a particle, crystallinity, measurement conditions, etc. Thus, it should not be rigidly understood.

In general, the value of wave number (cm⁻¹) in the attenuated total reflection infrared spectroscopy (ATR method) may cause an error in the range of ±2 cm⁻¹. Accordingly, the term "wave number Y cm⁻¹" is used in the present description to mean "wave number ((Y - 2) to (Y + 2)) cm⁻¹", unless otherwise specified.

Next, the compound of the present invention will be described.

The compound of the present invention is a compound represented by the formula [12]: 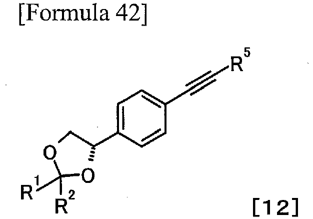 wherein R¹, R², and R⁵ are as defined above.

The compound of the present invention is novel compound which is not known so far.

R¹ is a C₁₋₃ alkyl group.

R¹ is preferably a methyl group or an ethyl group, more preferably a methyl group.

R² is a C₁₋₃ alkyl group.

R² is preferably a methyl group or an ethyl group, more preferably a methyl group.

Further preferably, R¹ is a methyl group and R² is a methyl group.

R⁵ is a hydrogen atom, a group represented by the formula [13]: 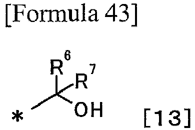
wherein R⁶, R⁷, and * are as defined above, or
a group represented by the formula [14]: 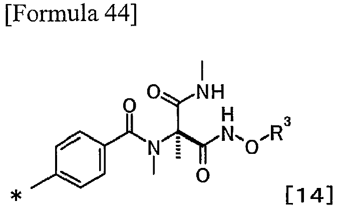
wherein R³ and * are as defined above.

R⁵ is preferably a hydrogen atom.

More preferably, R¹ is a methyl group, R² is a methyl group, and R⁵ is a hydrogen atom.

In another embodiment, R⁵ is preferably a group represented by the formula [13]: 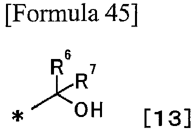
wherein R⁶, R⁷, and * are as defined above.
R⁶ is a C₁₋₃ alkyl group; and R⁷ is a C₁₋₃ alkyl group; or R⁶ and R⁷ together are a C₃₋₆ alkylene group.
R⁶ is preferably a methyl group.
R⁷ is preferably a methyl group.

More preferably, R⁶ is a methyl group and R⁷ is a methyl group.

Further preferably, R¹ is a methyl group, R² is a methyl group, R⁶ is a methyl group and R⁷ is a methyl group.

In another embodiment, R⁵ is preferably a group represented by the formula [14]: 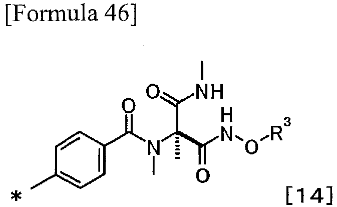
wherein R³ and * are as defined above.
R³ is a hydrogen atom or a hydroxyl-protecting group.
R³ is preferably a hydrogen atom.

More preferably, R¹ is a methyl group, R² is a methyl group, and R³ is a hydrogen atom.

In another embodiment, R³ is preferably a hydroxyl-protecting group, more preferably a tetrahydropyranyl group.

Further preferably, R¹ is a methyl group, R² is a methyl group, and R³ is a tetrahydropyranyl group.

In another embodiment, examples of the compound of the present invention include (4S)-4-(4-ethynylphenyl)-2,2-dimethyl-1,3-dioxolane, 4-(4-((4S)-2,2-dimethyl-1,3-dioxolan-4-yl)phenyl)-2-methyl-3-butyn-2-ol, (2S)-2-((4-((4-((4S)-2,2-dimethyl-1,3-dioxolan-4-yl)phenyl)ethynyl)benzoyl)(methyl)amino)-N,2-dimethyl-N'-(tetrahydro-2H-pyran-2-yloxy)malonamide, and (2S)-2-((4-((4-((4S)-2,2-dimethyl-1,3-dioxolan-4-yl)phenyl)ethynyl)benzoyl)(methyl)amino)-N-hydroxy-N',2-dimethylmalonamide.

Next, a compound used in the production method of the present invention will be described.

Preferred groups represented by R¹, R², R³, R⁵, R⁶, and R⁷ are as mentioned above.

R^{3a} is a hydroxyl-protecting group.

R^{3a} is preferably a tetrahydropyranyl group.

R⁴ is an optionally substituted acyl group.

R⁴ is preferably an acyl group, more preferably a C₂₋₆ alkanoyl group, further preferably an acetyl group.

Examples of the substituent for the acyl group represented by R⁴ include one or two or more groups selected from substituent group A.

L¹ is a leaving group.

L¹ is preferably a bromine atom or an iodine atom, more preferably an iodine atom.

Next, the production method of the present invention will be described.

### [Production method 1]

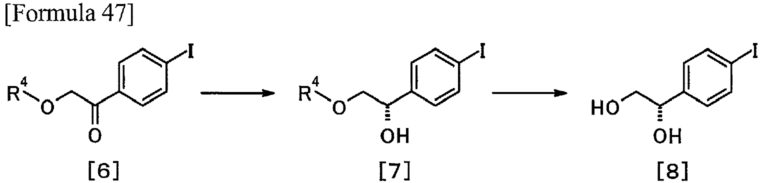

wherein R⁴ is as defined above.

For example, (2-(4-iodophenyl)-2-oxoethyl) acetate is known as the compound represented by the formula [6].

The compound represented by the formula [6] can be easily produced from a corresponding haloketone compound.

The compound represented by the formula [7] can be produced by subjecting the compound represented by the formula [6] to reduction reaction.
(1-1) Examples of the reduction reaction include hydrogenation reaction using a metal catalyst.

The solvent for use in this reaction is not particularly limited as long as the solvent has no adverse effect on the reaction. Examples thereof include aliphatic hydrocarbons, halogenated hydrocarbons, alcohols, ethers, ketones, esters, amides, nitriles, aromatic hydrocarbons, sulfoxides, and water. These solvents may be used as a mixture. Preferred examples of the solvent include amides. N,N-Dimethylformamide is more preferred.

Examples of the metal catalyst for use in this reaction include [(R,R)-N-(2-amino-1,2-diphenylethyl)-p-toluenesulfonamido]chloro(p-cymene)ruthenium(II), [(R,R)-N-(2-amino-1,2-diphenylethyl)-p-toluenesulfonamido]chloro(mesitylene)ruthenium(II ), [(R,R)-N-(2-amino-1,2-diphenylethyl)pentafluorobenzenesulfonamido]chloro(p-cymene)ruth enium(II), chloro[(R,R)-N-[2-[2-(4-methylbenzyloxy)ethyl]amino-1,2-diphenylethyl]-p-toluenesulfona mido]ruthenium(II), and chloro[(R,R)-N-[2-[2-(4-methylbenzyloxy)ethyl]amino-1,2-diphenylethyl]-methanesulfonami do]ruthenium(II). [(R,R)-N-(2-Amino-1,2-diphenylethyl)-p-toluenesulfonamido]chloro(p-cymene)ruthenium(II ) is preferred.

The amount of the metal catalyst used can be 0.001 to 1 times the mol of the compound represented by the formula [6] and is preferably 0.001 to 0.01 times the mol of the compound represented by the formula [6].

Examples of the reducing agent include: 2-propanol; formic acid; formates such as sodium formate, ammonium formate, and triethylammonium formate; and cyclohexene and cyclohexadiene. Formic acid is preferred.

The amount of the reducing agent used can be 2 to 100 times the mol of the compound represented by the formula [6] and is preferably 2 to 10 times the mol of the compound represented by the formula [6].

For this reaction, it is preferred to add a base. Examples of the base used include inorganic bases or organic bases. An organic base is preferred.

The amount of the base used can be 0.5 to 50 times the mol of the compound represented by the formula [6] and is preferably 2 to 5 times the mol of the compound represented by the formula [6].

In this step, the reaction is particularly preferably performed in the presence of an organic base using [(R,R)-N-(2-amino-1,2-diphenylethyl)-p-toluenesulfonamido]chloro(p-cymene)ruthenium(II) as the metal catalyst and formic acid as the reducing agent. Use of [(R,R)-N-(2-amino-1,2-diphenylethyl)-p-toluenesulfonamido]chloro(p-cymene)ruthenium(II) as the metal catalyst can circumvent use of hydrogen gas and permits safe reaction.

This reaction can be carried out at -50 to 200°C, preferably 10 to 50°C, for 10 minutes to 48 hours.

This reaction can be preferably carried out in an inert gas (e.g., nitrogen or argon) atmosphere.

The compound represented by the formula [7] may be isolated or may be used directly in the next reaction without being isolated.
(1-2) Also, the compound represented by the formula [7] can be produced by subjecting the compound represented by the formula [6] to reduction reaction using borane or a borane complex in the presence of oxazaborolidine.

The solvent for use in this reaction is not particularly limited as long as the solvent has no adverse effect on the reaction. Examples thereof include halogenated hydrocarbons, ethers, and aromatic hydrocarbons. These solvents may be used as a mixture. Preferred examples of the solvent include ethers. Tetrahydrofuran is more preferred.

Examples of the oxazaborolidine for use in this reaction include
(S)-5,5-diphenyl-3,4-propano-1,3,2-oxazaborolidine,
(S)-5,5-diphenyl-2-methyl-3,4-propano-1,3,2-oxazaborolidine,
(S)-5,5-diphenyl-2-butyl-3,4-propano-1,3,2-oxazaborolidine, and
(S)-3,3-diphenyl-1-o-tolyl-tetrahydropyrrolo(1,2,c)(1,3,2)oxazaborole.
(S)-5,5-Diphenyl-2-methyl-3,4-propano-1,3,2-oxazaborolidine is preferred.

The amount of the oxazaborolidine used can be 0.001 to 1 times the mol of the compound represented by the formula [6] and is preferably 0.01 to 0.1 times the mol of the compound represented by the formula [6].

The borane or the borane complex for use in this reaction is preferably a borane complex.

Examples of the borane complex for use in this reaction include a borane-tetrahydrofuran complex, a borane-dimethyl sulfide complex, a borane-1,4-dioxane complex, a borane-diethylaniline complex, and catecholborane. A borane-diethylaniline complex is preferred.

The amount of the borane or the borane complex used can be 1 to 10 times the mol of the compound represented by the formula [6] and is preferably 1 to 5 times the mol of the compound represented by the formula [6].

This reaction can be carried out at -78 to 50°C, preferably 10 to 50°C, for 10 minutes to 48 hours.

This reaction can be preferably carried out in an inert gas (e.g., nitrogen or argon) atmosphere.

The compound represented by the formula [7] may be isolated or may be used directly in the next reaction without being isolated.
(2) The compound represented by the formula [8] can be produced by subjecting the compound represented by the formula [7] to deprotection reaction.

This reaction can be performed by, for example, a method described in Greene's Protective Groups in Organic Synthesis, 5th ed., p. 17-471, 2014, John Wiley & Sons, INC.

The compound represented by the formula [8] may be isolated or may be used directly in the next reaction without being isolated.

### [Production method 2]

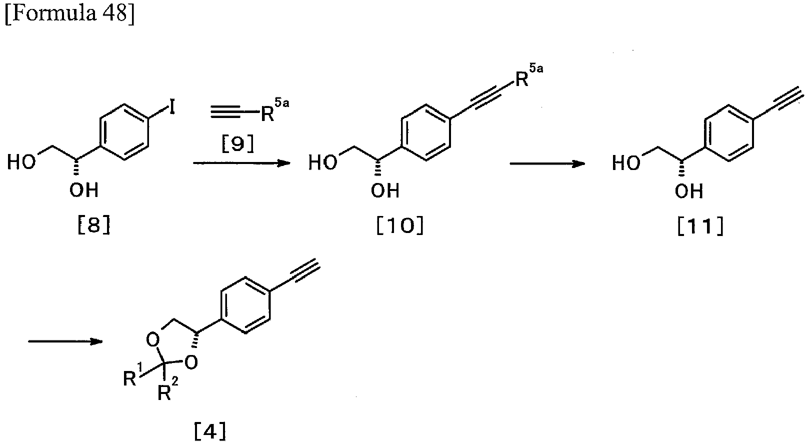

wherein R¹, R², and R^{5a} are as defined above.
(1) For example, trimethylsilylacetylene is known as the compound represented by the formula [9].

The compound represented by the formula [10] can be produced by reacting the compound represented by the formula [8] with the compound represented by the formula [9] in the presence or absence of a base, in the presence or absence of a copper catalyst, in the presence or absence of a ligand, and in the presence of a palladium catalyst.

Since the compound represented by the formula [8] has an iodo group at position 4 of the phenyl group, the compound represented by the formula [10] can be produced by reacting the compound represented by the formula [8] with the compound represented by the formula [9] under mild conditions.

This reaction can be performed by a method described in, for example, International Publication No. WO 2014/142298 or a method equivalent thereto.

The solvent for use in this reaction is not particularly limited as long as the solvent has no adverse effect on the reaction. Examples thereof include aliphatic hydrocarbons, halogenated hydrocarbons, alcohols, ethers, ketones, esters, amides, nitriles, aromatic hydrocarbons, sulfoxides, and water. These solvents may be used as a mixture. Preferred examples of the solvent include ethers. Tetrahydrofuran is more preferred.

Examples of the base used, if desired, in this reaction include organic bases and inorganic bases. Preferred examples of the base include organic bases. Triethylamine is more preferred.

The amount of the base used can be 0.5 to 50 times the mol of the compound represented by the formula [8] and is preferably 2 to 5 times the mol of the compound represented by the formula [8].

Examples of the copper catalyst used, if desired, in this reaction include copper(I) bromide and copper(I) iodide. Copper(I) iodide is preferred.

The amount of the copper catalyst used can be 0.01 to 50 times the mol of the compound represented by the formula [8] and is preferably 0.01 to 0.2 times the mol of the compound represented by the formula [8].

Examples of the ligand used, if desired, in this reaction include tri-tert-butylphosphine, tricyclohexylphosphine, triphenylphosphine, tritolylphosphine, tributyl phosphite, tricyclohexyl phosphite, triphenyl phosphite, 1,1'-bis(diphenylphosphino)ferrocene, 2,2'-bis(diphenylphosphino)-1,1'-binaphthyl, 2-dicyclohexylphosphino-2',6'-dimethoxybiphenyl, 2-dicyclohexylphosphino-2',4',6'-triisopropylbiphenyl, 2-(di-t-butylphosphino)-2',4',6'-triisopropylbiphenyl, 2-(dicyclohexylphosphino)-3,6-dimethoxy-2',4',6'-triisopropyl-1,1'-biphenyl, and 2-(di-t-butylphosphino)biphenyl. These ligands may be used in combination.

The amount of the ligand used can be 0.00001 to 1 times the mol of the compound represented by the formula [8] and is preferably 0.001 to 0.1 times the mol of the compound represented by the formula [8].

Examples of the palladium catalyst for use in this reaction include: metal palladium such as palladium-carbon and palladium black; inorganic palladium salts such as palladium chloride and sodium palladium(II) chloride trihydrate; organic palladium salts such as palladium acetate; organic palladium complexes such as tetrakis(triphenylphosphine)palladium(0), bis(triphenylphosphine)palladium(II) dichloride, bis(acetonitrile)palladium(II) dichloride, bis(benzonitrile)palladium(II) dichloride, 1,1'-bis(diphenylphosphino)ferrocene palladium(II) dichloride, tris(dibenzylideneacetone)dipalladium(0), bis(dibenzylideneacetone)palladium(0), bis(tricyclohexylphosphine)palladium(II) dichloride, bis(tri-o-tolylphosphine)palladium(II) dichloride, bis(tri-tert-butylphosphine)palladium(II) dichloride, (1,3-bis(2,6-diisopropylphenyl)imidazolidene)(3-chloropyridyl)palladium(II) dichloride, allyl palladium(II) chloride dimers, and bis(di-tert-butyl(4-dimethylaminophenyl)phosphine)palladium(II) dichloride; polymer-immobilized organic palladium complexes such as polymer-supported bis(acetato)triphenylphosphine palladium(II) and polymer-supported di(acetato)dicyclohexylphenylphosphine palladium(II); and palladium-NHC complexes such as bis(1,4-naphthoquinone)bis[1,3-bis(2,6-diisopropylphenyl)imidazol-2-ylidene]dipalladium(0). These palladium catalysts may be used in combination. Preferred examples of the palladium catalyst include tetrakis(triphenylphosphine)palladium(0), bis(1,4-naphthoquinone)bis[1,3-bis(2,6-diisopropylphenyl)imidazol-2-ylidene]dipalladium(0), and bis(triphenylphosphine)palladium(II) dichloride. Bis(triphenylphosphine)palladium(II) dichloride is more preferred.

The amount of the palladium catalyst used can be 0.00001 to 1 times the mol of the compound represented by the formula [8] and is preferably 0.001 to 0.1 times the mol of the compound represented by the formula [8].

The amount of the compound represented by the formula [9] used can be 0.5 to 50 times the mol of the compound represented by the formula [8] and is preferably 0.8 to 5 times the mol of the compound represented by the formula [8].

In this step, trimethylsilylacetylene is particularly preferably used as the compound represented by the formula [9]. Use of trimethylsilylacetylene permits deprotection reaction of the compound represented by the formula [10] under mild conditions.

This reaction can be carried out at -50 to 200°C, preferably 10 to 50°C, for 10 minutes to 48 hours.

This reaction can be preferably carried out in an inert gas (e.g., nitrogen or argon) atmosphere.

The compound represented by the formula [10] may be isolated or may be used directly in the next reaction without being isolated.
(2) The compound represented by formula [11] can be produced by subjecting the compound represented by the formula [10] to deprotection reaction.

This reaction can be performed by, for example, a method described in Greene's Protective Groups in Organic Synthesis, 5th ed., p. 1194-1202, 2014, John Wiley & Sons, INC.

The compound represented by formula [11] may be isolated or may be used directly in the next reaction without being isolated.
(3) The compound represented by the formula [4] can be produced by protecting the diol group in the compound represented by formula [11].

This reaction can be performed by, for example, a method described in Greene's Protective Groups in Organic Synthesis, 5th ed., p. 17-471, 2014, John Wiley & Sons, INC.

Specifically, the compound represented by the formula [4] can be produced by reacting the compound represented by formula [11] with a compound represented by the formula [16]: 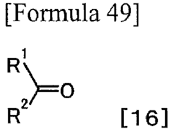
wherein R¹ and R² are as defined above, or
a compound represented by the formula [17]: 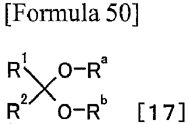
wherein R^{a} represents a C₁₋₃ alkyl group; R^{b} represents a C₁₋₃ alkyl group; and R¹ and R² are as defined above
in the presence of an acid catalyst.

The solvent for use in this reaction is not particularly limited as long as the solvent has no adverse effect on the reaction. Examples thereof include aliphatic hydrocarbons, halogenated hydrocarbons, alcohols, ethers, ketones, esters, amides, nitriles, aromatic hydrocarbons, sulfoxides, and water. These solvents may be used as a mixture. Preferred examples of the solvent include amides. N-Methylpyrrolidone is more preferred.

Examples of the acid for use in this reaction include organic acids and inorganic acids. Preferred examples of the acid include organic acids. An organic sulfonic acid is more preferred, and methanesulfonic acid is further preferred.

The amount of the acid used can be 0.01 to 1 times the mol of the compound represented by formula [11] and is preferably 0.05 to 0.2 times the mol of the compound represented by formula [11].

The amount of the compound represented by the formula [16] or the compound represented by the formula [17] used can be 0.5 to 50 times the mol of the compound represented by formula [11] and is preferably 1 to 5 times the mol of the compound represented by formula [11].

In this step, the compound represented by the formula [17] is preferably used, and 2,2-dimethoxypropane is particularly preferably used as the compound represented by the formula [17].

This reaction can be carried out at -50 to 200°C, preferably 10 to 50°C, for 10 minutes to 48 hours.

This reaction can be preferably carried out in an inert gas (e.g., nitrogen or argon) atmosphere.

The compound represented by the formula [4] may be isolated or may be used directly in the next reaction without being isolated.

The compound represented by the formula [4] can also be produced by the following production method.

### [Production method 3]

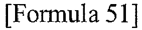

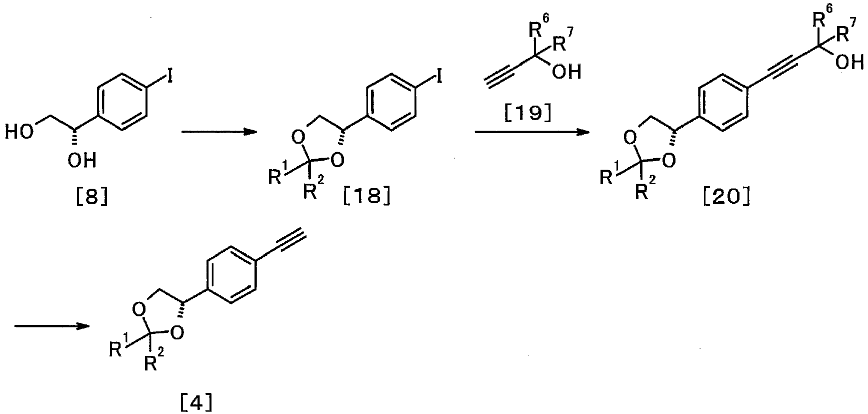

wherein R¹, R², R⁶, and R⁷ are as defined above.
(1) The compound represented by the formula [18] can be produced by protecting the diol group in the compound represented by the formula [8].
   This reaction can be performed according to the method described in production method 2(3).
(2) For example, 2-methyl-3-butyn-2-ol is known as the compound represented by the formula [19].
   The compound represented by the formula [20] can be produced by reacting the compound represented by the formula [18] with the compound represented by the formula [19].
   This reaction can be performed according to the method described in production method 2(1).
(3) The compound represented by the formula [4] can be produced by subjecting the compound represented by the formula [20] to deprotection reaction.

This reaction can be performed by, for example, a method described in Greene's Protective Groups in Organic Synthesis, 5th ed., p. 1194-1202, 2014, John Wiley & Sons, INC.

### [Production method 4]

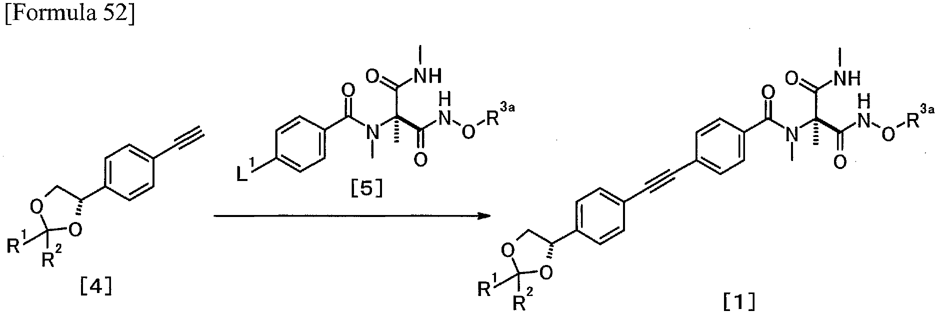

wherein R¹, R², R^{3a}, and L¹ are as defined above.

The compound represented by the formula [1] can be produced by reacting the compound represented by the formula [4] with the compound represented by the formula [5] in the presence or absence of a base, in the presence or absence of a copper catalyst, in the presence or absence of a ligand, and in the presence of a palladium catalyst.

For example, (2S)-2-((4-iodobenzoyl)(methyl)amino)-N,2-dimethyl-N'-(tetrahydro-2H-pyran-2-yloxy)malo namide is known as the compound of the formula [5].

This reaction can be performed according to the method described in production method 2(1).

### [Production method 5]

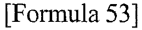

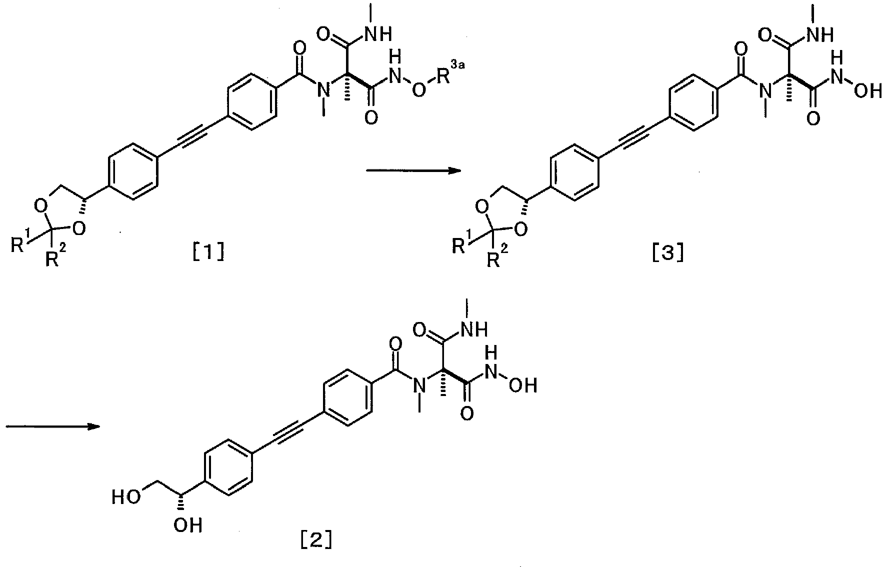

wherein R¹, R², and R^{3a} are as defined above.
(1) The compound represented by the formula [3] can be produced by deprotecting the hydroxyl-protecting group in the compound represented by the formula [1]. This reaction can be performed by, for example, a method described in Greene's Protective Groups in Organic Synthesis, 5th ed., p. 17-471, 2014, John Wiley & Sons, INC.
   When R^{3a} is, for example, a tetrahydropyranyl group, the compound represented by the formula [3] can be produced by subjecting the compound represented by the formula [1] to deprotection reaction using an acid.
   The solvent for use in this reaction is not particularly limited as long as the solvent has no adverse effect on the reaction. Examples thereof include aliphatic hydrocarbons, halogenated hydrocarbons, alcohols, ethers, ketones, esters, amides, nitriles, aromatic hydrocarbons, sulfoxides, and water. These solvents may be used as a mixture. Preferred examples of the solvent include alcohols, ethers, ketones, and amides. Alcohols and ketones are more preferred.
   Examples of the acid for use in this reaction include organic acids and inorganic acids. Preferred examples of the acid include organic acids. Methanesulfonic acid is more preferred.
   The amount of the acid used can be 0.01 to 1.0 times the mol of the compound represented by the formula [3] and is preferably 0.1 to 0.5 times the mol of the compound represented by the formula [3].
   This reaction can be carried out at -50 to 200°C, preferably -10 to 10°C, for 10 minutes to 48 hours.
(2) The compound represented by formula [2] can be produced by deprotecting the compound represented by the formula [3] using an acid. This reaction can be performed by, for example, a method described in Greene's Protective Groups in Organic Synthesis, 5th ed., p. 17-471, 2014, John Wiley & Sons, INC.

The solvent for use in this reaction is not particularly limited as long as the solvent has no adverse effect on the reaction. Examples thereof include aliphatic hydrocarbons, halogenated hydrocarbons, alcohols, ethers, ketones, esters, amides, nitriles, aromatic hydrocarbons, sulfoxides, and water. These solvents may be used as a mixture. Preferred examples of the solvent include alcohols, ethers, ketones, amides, and nitriles. Alcohols and nitriles are more preferred.

Examples of the acid for use in this reaction include organic acids and inorganic acids. Preferred examples of the acid include inorganic acids. Hydrochloric acid is more preferred.

The amount of the acid used can be 0.1 to 10 times the mol of the compound represented by the formula [3] and is preferably 1 to 3 times the mol of the compound represented by the formula [3].

This reaction can be carried out at -50 to 200°C, preferably 0 to 20°C, for 10 minutes to 48 hours.

For the compound represented by the formula [1], it is preferred that the diol group-protecting group should not be deprotected under conditions where the hydroxyl-protecting group represented by R^{3a} is deprotected.

Particularly preferably, R¹ is a methyl group, R² is a methyl group, and R^{3a} is a tetrahydropyranyl group.

The tetrahydropyranyl group represented by R^{3a} is deprotected under conditions where the diol group-protecting group is not deprotected.

In the step of deprotecting the compound represented by the formula [1] to produce the compound represented by formula [2], as shown above, the compound represented by the formula [3] may be isolated, or the compound represented by the formula [3] may not be isolated.

In order to produce the compound represented by the formula [2] having high purity, the compound represented by the formula [3] is preferably isolated.

The compound represented by formula [2] obtained by the production methods described above has high optical purity. The optical purity of the compound represented by formula [2] can be measured by, for example, a routine method using a chiral column.

When each compound for use in the production methods described above has a solvate, a hydrate, and crystals in various forms, the solvate, the hydrate, and the crystals in various forms can also be used.

When each compound for use in the production methods described above is, for example, a compound having an amino group, a hydroxyl group, or a carboxyl group, etc., these groups can be protected in advance with usual protecting groups and these protecting groups can be eliminated by methods known per se in the art after reaction.

Each compound obtained by the production methods described above can be converted to a different compound through reaction known per se in the art, for example, condensation, addition, oxidation, reduction, translocation, substitution, halogenation, dehydration, or hydrolysis, or by an appropriate combination of these reactions.

### Examples

Next, the present invention will be described with reference to Examples. However, the present invention is not intended to be limited by these examples.

Unless otherwise specified, silica gel column chromatography is flash column chromatography, and a carrier therefor is silica gel 60 (Kanto Chemical Co., Inc.).

A mixing ration in an eluent is a volume ratio. For example, "gradient elution of hexane:ethyl acetate = 100:0 → 50:50" means that an eluent of 100% hexane/0% ethyl acetate was finally changed to an eluent of 50% hexane/50% ethyl acetate.

In NMR spectra, for example, the term "[1.81],1.82(3H,s)" means that peaks derived from diastereomers in a diastereomer mixture were observed as singlets at 1.81 ppm and 1.82 ppm, respectively, and the total number of protons was 3H.

Using Ultima IV (Rigaku Corporation), the powder X-ray diffraction was measured under the following conditions.

### Measurement conditions

Used X-ray: CuKα
Tube voltage: 40 kV
Tube current: 40 mA
Scanning axis: 2θ

The water content was measured using Karl Fischer moisture meter CA-100 (Mitsubishi Chemical Corporation).

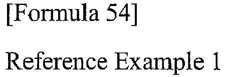

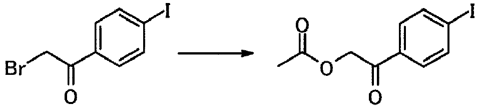

To a mixture of 2-bromo-1-(4-iodophenyl)ethanone (10.00 g), ethanol (39 mL), sodium acetate (3.89 g), and water (19.5 mL), acetic acid (2.03 g) was added in a nitrogen atmosphere, and the resulting mixture was stirred at 70 to 75°C for 3 hours. The reaction mixture was cooled to room temperature. Then, water (19.5 mL) was added thereto, and the mixture was stirred at the same temperature as above for 1 hours. Solid matter was collected by filtration and washed with water (27 mL) twice to obtain 8.92 g of (2-(4-iodophenyl)-2-oxoethyl) acetate as a pale yellow solid.
¹H-NMR (600 MHz, CDCl₃) δ: 2.23 (3H, s), 5.28 (2H, s), 7.62 (2H, d, J = 9.0 Hz), 7.86 (2H, d, J = 9.0 Hz).

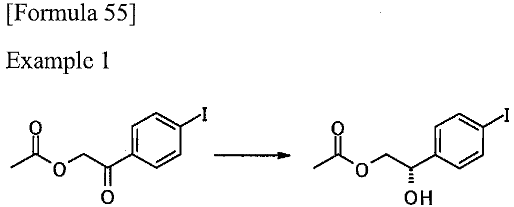

To a mixture of N,N-dimethylformamide (5.0 mL) and N,N-diisopropylethylamine (1.15 mL), formic acid (0.62 mL), (2-(4-iodophenyl)-2-oxoethyl) acetate (1.00 g), and [(R,R)-N-(2-amino-1,2-diphenylethyl)-p-toluenesulfonamido]chloro(p-cymene)ruthenium(II) (10.5 mg) were added in this order at 0 to 10°C in a nitrogen atmosphere, and the resulting mixture was stirred at 20 to 30°C for 6.5 hours. Ethyl acetate (10 mL) and a 20% aqueous sodium chloride solution (10 mL) were added to the reaction mixture. The organic layer was separated and washed with a 20% aqueous sodium chloride solution (5 mL) once, a 5% aqueous sodium bicarbonate solution (5 mL) twice, and a 20% aqueous sodium chloride solution (5 mL) once. Active carbon (0.10 g) was added to the obtained organic layer, and the mixture was stirred at 20 to 30°C for 40 minutes and dried over anhydrous magnesium sulfate. Then, the solvent was distilled off under reduced pressure to obtain 0.99 g of ((2S)-2-hydroxy-2-(4-iodophenyl)ethyl) acetate as a pale yellow oil.
¹H-NMR (600 MHz, CDCl₃) δ: 2.10 (3H, s), 2.72 (1H, s), 4.10 (1H, dd, J = 11.4, 7.8 Hz), 4.24 (1H, dd, J = 12.0, 3.6 Hz), 4.90 (1H, d, J = 7.8 Hz), 7.14 (2H, d, J = 8.4 Hz), 7.70 (2H, d, J = 8.4 Hz).

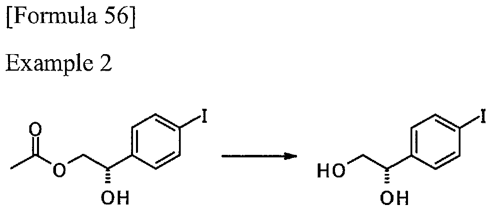

To a mixture of ((2S)-2-hydroxy-2-(4-iodophenyl)ethyl) acetate (0.48 g) and methanol (2.5 mL), potassium carbonate (0.34 g) was added in a nitrogen atmosphere, and the resulting mixture was stirred at room temperature for 2 hours. Methylene chloride (10 mL), water (5 mL), a saturated aqueous solution of sodium chloride (5 mL), and 2-methyl-2-propanol (0.1 mL) were added to the reaction mixture. The organic layer was separated, and the aqueous layer was subjected to extraction with a mixed solution of methylene chloride (10 mL) and 2-methyl-2-propanol (0.1 mL). The organic layer and the extract were combined and dried over anhydrous sodium sulfate. Then, the solvent was distilled off under reduced pressure. Diisopropyl ether was added to the obtained residue, and solid matter was collected by filtration to obtain 0.30 g of (1S)-1-(4-iodophenyl)ethane-1,2-diol as a pale yellow solid.

The obtained solid was used as seed crystals in Example 3.

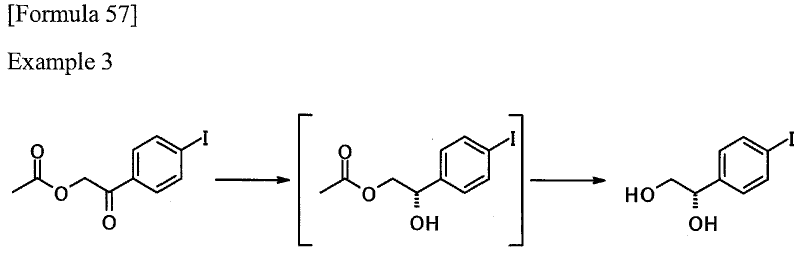

To a mixture of N,N-dimethylformamide (100 mL) and N,N-diisopropylethylamine (17.0 g), formic acid (15.1 g), (2-(4-iodophenyl)-2-oxoethyl) acetate (20.0 g), and [(R,R)-N-(2-amino-1,2-diphenylethyl)-p-toluenesulfonamido]chloro(p-cymene)ruthenium(II) (0.21 g) were added in this order at 0 to 10°C in a nitrogen atmosphere, and the resulting mixture was stirred at 15 to 20°C for 22.5 hours. Ethyl acetate (300 mL) and a 20% aqueous sodium chloride solution (200 mL) were added to the reaction mixture. The organic layer was separated and washed with a 20% aqueous sodium chloride solution (100 mL) once, a 5% aqueous sodium bicarbonate solution (100 mL) twice, and a 20% aqueous sodium chloride solution (100 mL) once. Active carbon (2.00 g) was added to the obtained organic layer, and the mixture was stirred at 20 to 30°C for 1 hour. Insoluble matter was filtered off, and the solvent was distilled off under reduced pressure to obtain ((2S)-2-hydroxy-2-(4-iodophenyl)ethyl) acetate as a pale yellow oil.

To a mixture of the obtained ((2S)-2-hydroxy-2-(4-iodophenyl)ethyl) acetate and methanol (100 mL), potassium carbonate (13.64 g) was added in a nitrogen atmosphere, and the resulting mixture was stirred at 20 to 30°C for 1.5 hours. Insoluble matter was filtered off, and the solvent was distilled off under reduced pressure. Methanol (100 mL) and water (70 mL) were added to the obtained residue. After confirmation of dissolution, the seed crystals (0.05 g) were added to the solution, and the mixture was stirred at 20 to 30°C for 2 hours. Water (10 mL) and 6 mol/L hydrochloric acid (11.3 mL) were added to the reaction mixture for pH adjustment to 7 to 8, and the mixture was then stirred at 20 to 30°C for 2 hours and left standing overnight. Water (100 mL) was added to the reaction mixture over 1 hour, and the mixture was stirred at the same temperature as above for 2.5 hours. Water (100 mL) was added to the reaction mixture over 2 hours, and the mixture was stirred at the same temperature as above for 1.5 hours. Water (220 mL) was added to the reaction mixture over 1.5 hours, and the mixture was stirred at the same temperature as above for 1 hour. The reaction mixture was cooled to 0 to 10°C and then stirred at the same temperature as above for 1 hour. Solid matter was collected by filtration and washed with a 10% aqueous methanol solution (40 mL) twice to obtain 15.4 g of (1S)-1-(4-iodophenyl)ethane-1,2-diol as a pale yellow solid.
Optical purity: 96.2% ee

### HPLC measurement conditions

Measurement wavelength: 230 nm
Column: CHIRALPAK ID (Daicel Corporation), particle diameter: 5 µm, inner diameter 4.6 mm × length 250 mm
Column temperature: 40°C
Flow rate: 1.0 mL/min
Mobile phase: hexane/ethanol = 970/30
¹H-NMR (600 MHz, DMSO-d₆) δ: 3.35-3.44 (2H, m), 4.48 (1H, dd, J = 11.4, 5.4 Hz), 4.73 (1H, t, J = 6.6 Hz), 5.31 (1H, d, J = 4.2 Hz), 7.15 (2H, d, J = 7.8 Hz), 7.66 (2H, d, J = 8.4 Hz).

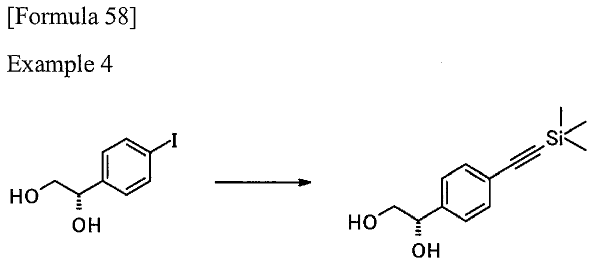

To a mixture of (1S)-1-(4-iodophenyl)ethane-1,2-diol (10.0 g), bis(triphenylphosphine)palladium(II) dichloride (0.13 g), copper(I) iodide (0.11 g), triethylamine (9.96 g), and tetrahydrofuran (50 mL), a mixture of trimethylsilylacetylene (4.09 g) and tetrahydrofuran (20 mL) was added over 1 hour in a nitrogen atmosphere, and the resulting mixture was stirred at room temperature for 1 hour. Ethyl acetate (100 mL) and a 5% aqueous ammonium sulfate solution (50 mL) were added to the reaction mixture. The organic layer was separated and washed with a 40% aqueous ammonium sulfate solution (50 mL), a 10% aqueous N-acetylcysteine solution (50 mL), a 5% aqueous sodium bicarbonate solution (50 mL), and a mixed solution of a 5% aqueous sodium bicarbonate solution and a 20% aqueous sodium chloride solution (50 mL) in this order. Anhydrous sodium sulfate (20 g) and SH SILICA (1.0 g) were added to the obtained organic layer, and the mixture was stirred at room temperature for 30 minutes. Active carbon (0.5 g) was added to the obtained mixture, and the resulting mixture was stirred at room temperature. Insoluble matter was filtered off, and the solvent was distilled off under reduced pressure. Heptane and isopropyl acetate were added to the obtained residue, and solid matter was collected by filtration and washed with a mixed solution of heptane and isopropyl acetate to obtain 7.10 g of (1S)-1-(4-((trimethylsilyl)ethynyl)phenyl)ethane-1,2-diol as a white solid.
¹H-NMR (600 MHz, CDCl₃) δ: 0.25 (9H, s), 2.42-2.49 (1H, m), 2.92-3.00 (1H, m), 3.54-3.63 (1H, m), 3.67-3.75 (1H, m), 4.74-4.81 (1H, m), 7.28 (2H, d, J = 8.4 Hz), 7.45 (2H, d, J = 8.4 Hz).

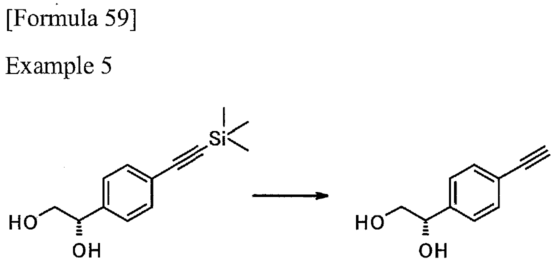

To a mixture of (1S)-1-(4-((trimethylsilyl)ethynyl)phenyl)ethane-1,2-diol (0.36 g) and methanol (3 mL), potassium carbonate (0.24 g) was added in a nitrogen atmosphere, and the resulting mixture was stirred at room temperature for 1.5 hours. Water (3 mL) was added to the reaction mixture, and the mixture was stirred at room temperature for 1 hour. Insoluble matter was filtered off, and methylene chloride was added to the filtrate. The organic layer was separated, and the aqueous layer was subjected to extraction with methylene chloride three times. The organic layer and the extracts were combined and dried over anhydrous sodium sulfate. Then, the solvent was distilled off under reduced pressure. Hexane and isopropyl acetate were added to the obtained residue, and solid matter was collected by filtration to obtain 0.14 g of (1S)-1-(4-ethynylphenyl)ethane-1,2-diol as a pale yellow solid.

The obtained solid was used as seed crystals in Example 6.

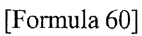

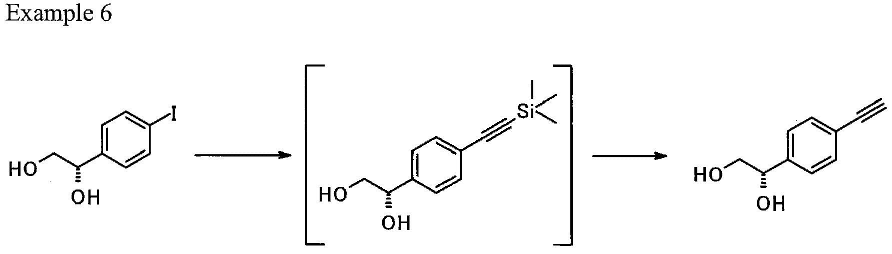

(1S)-1-(4-((Trimethylsilyl)ethynyl)phenyl)ethane-1,2-diol was obtained as a pale yellow solid in the same way as in Example 4 using (1S)-1-(4-iodophenyl)ethane-1,2-diol (5.00 g).

To a mixture of the obtained (1S)-1-(4-((trimethylsilyl)ethynyl)phenyl)ethane-1,2-diol and methanol (25 mL), potassium carbonate (3.93 g) was added in a nitrogen atmosphere, and the resulting mixture was stirred at 20 to 30°C for 1.5 hours. Active carbon (0.50 g) was added to the reaction mixture, and the mixture was stirred at 20 to 30°C for 1 hour. Insoluble matter was filtered off, and the solvent was distilled off under reduced pressure. Methanol (6.2 mL) and water (6.2 mL) were added to the obtained residue at 20 to 30°C. After confirmation of dissolution, 6 mol/L hydrochloric acid was added to the solution for pH adjustment to 6 to 7. Water (7.8 mL) and a 25% aqueous sodium chloride solution (6.2 mL) were added to the obtained mixture at the same temperature as above, then the seed crystals were added thereto, and the resulting mixture was then stirred at the same temperature as above for 1.5 hours. A 25% aqueous sodium chloride solution (6.2 mL) was added to the obtained mixture, and the resulting mixture was then stirred at 20 to 30°C for 1 hour. A 25% aqueous sodium chloride solution (6.2 mL) was added to the obtained mixture, and the resulting mixture was stirred at the same temperature as above for 4 hours and left standing overnight. The obtained mixture was stirred at 0 to 10°C for 1.5 hours. Solid matter was collected by filtration and washed with cold water (6.2 mL) twice to obtain 2.63 g of (1S)-1-(4-ethynylphenyl)ethane-1,2-diol as a pale yellow solid.
Optical purity: >99.9% ee

### HPLC measurement conditions

Measurement wavelength: 230 nm
Column: CHIRALPAK ID (Daicel Corporation), particle diameter: 5 µm, inner diameter 4.6 mm × length 250 mm
Column temperature: 40°C
Flow rate: 1.0 mL/min
Mobile phase: hexane/ethanol = 970/30

To a mixture of the obtained (1S)-1-(4-ethynylphenyl)ethane-1,2-diol (2.00 g) and ethyl acetate (40 mL), active carbon (0.20 g) was added in a nitrogen atmosphere, and the resulting mixture was stirred at 20 to 30°C for 1 hour. Insoluble matter was filtered off, and the solvent was distilled off under reduced pressure. Ethyl acetate (10 mL) was added to the obtained residue. After confirmation of dissolution, heptane (10 mL) was added to the solution, then the seed crystals were added thereto, and the mixture was then stirred at 20 to 30°C for 3 hours and left standing overnight. Heptane (15 mL) was added to the obtained mixture over 1 hour, and the resulting mixture was stirred at 20 to 30°C for 1 hour. Heptane (15 mL) was added to the obtained mixture over 1 hour, and the resulting mixture was stirred at the same temperature as above for 1 hour. The obtained mixture was cooled to 0 to 10°C and then stirred at the same temperature as above for 2 hours. Solid matter was collected by filtration and washed with heptane to obtain 1.67 g of (1S)-1-(4-ethynylphenyl)ethane-1,2-diol as a white solid.
¹H-NMR (400 MHz, DMSO-d₆) δ: 3.37-3.49 (2H, m), 4.11 (1H, s), 4.55 (1H, dd, J = 10.4, 5.6 Hz), 4.74 (1H, t, J = 5.6 Hz), 5.33 (1H, d, J = 4.4 Hz), 7.35 (2H, d, J = 8.4 Hz), 7.42 (2H, d, J = 8.0 Hz).

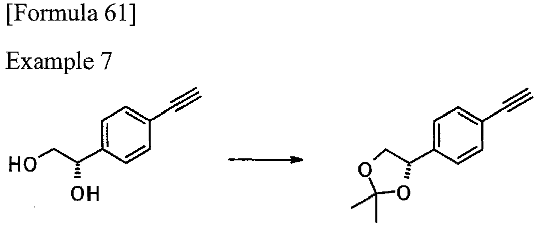

To a mixture of (1S)-1-(4-ethynylphenyl)ethane-1,2-diol (5.00 g), methylene chloride (50 mL), and 2,2-dimethoxypropane (19 mL), p-toluenesulfonic acid monohydrate (1.17 g) was added, and the resulting mixture was stirred at room temperature for 2 hours. 2,2-Dimethoxypropane (3.8 mL) was added to the reaction mixture, and the mixture was stirred at room temperature for 1 hour. Then, 2,2-dimethoxypropane (15 mL) was further added thereto, and the mixture was stirred at room temperature for 1 hour. A saturated aqueous solution of sodium bicarbonate (50 mL), water (20 mL), and methylene chloride were added to the reaction mixture. The organic layer was separated and washed with a saturated aqueous solution of sodium chloride (50 mL), and the aqueous layer was subjected to extraction with methylene chloride. The organic layer and the extract were combined and dried over anhydrous magnesium sulfate, and the solvent was distilled off under reduced pressure. The obtained residue was purified by silica gel column chromatography [eluent: hexane:ethyl acetate = 8:1] to obtain 6.03 g of (4S)-4-(4-ethynylphenyl)-2,2-dimethyl-1,3-dioxolane as a pale yellow oil.
¹H-NMR (400 MHz, CDCl₃) δ: 1.49 (3H, s), 1.55 (3H, s), 3.08 (1H, s), 3.67 (1H, t, J = 8.4 Hz), 4.31 (1H, dd, J = 8.4, 6.4 Hz), 5.07 (1H, dd, J = 7.6, 6.4 Hz), 7.33 (2H, d, J = 8.4 Hz), 7.49 (2H, d, J = 8.0 Hz).

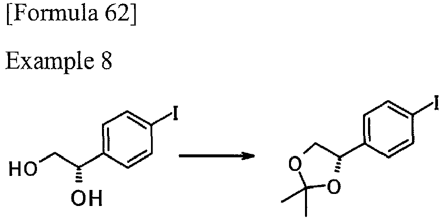

To a mixture of (1S)-1-(4-iodophenyl)ethane-1,2-diol (1.00 g), N-methylpyrrolidone (3 mL), and 2,2-dimethoxypropane (1.2 mL), methanesulfonic acid (25 µL) was added, and the resulting mixture was stirred at room temperature for 8 hours. Isopropyl acetate, a 20% aqueous sodium chloride solution, a 5% aqueous sodium bicarbonate solution, and water were added to the reaction solution. The organic layer was separated, washed with water, and dried over anhydrous sodium sulfate, and the solvent was distilled off under reduced pressure to obtain 1.35 g of (4S)-4-(4-iodophenyl)-2,2-dimethyl-1,3-dioxolane as a pale yellow oil.
¹H-NMR (400 MHz, CDCl₃) δ: 1.48 (3H, s), 1.53 (3H, s), 3.65 (1H, t, J = 8.0 Hz), 4.30 (1H, dd, J = 8.3, 6.1 Hz), 5.02 (1H, dd, J = 7.7, 6.5 Hz), 7.10-7.13 (2H, m), 7.60-7.70 (2H, m).

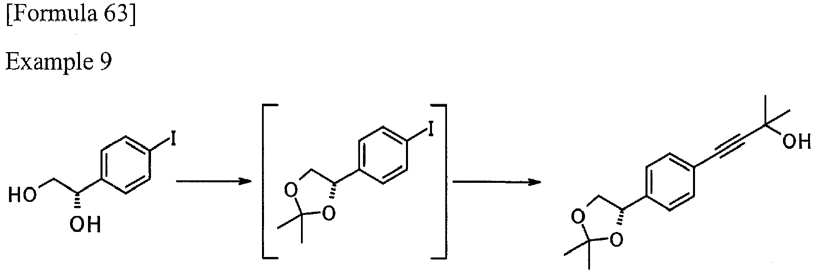

To a mixture of (1S)-1-(4-iodophenyl)ethane-1,2-diol (20 g), N-methylpyrrolidone (60 mL), and 2,2-dimethoxypropane (19.7 g), methanesulfonic acid (0.49 mL) was added, and the resulting mixture was stirred at room temperature for 3 hours. Triethylamine (23.0 g), bis(triphenylphosphine)palladium(II) dichloride (0.40 g), and copper(I) iodide (0.22 g) were added to the reaction mixture, then 3-methyl-1-butyn-3-ol (7.00 g) and N-methylpyrrolidone (5 mL) were added thereto at 25 to 40°C over 30 minutes in a nitrogen atmosphere, and the mixture was stirred at 30 to 38°C for 2 hours and 40 minutes. The reaction mixture was cooled to room temperature, and isopropyl acetate (100 mL) and a 40% aqueous ammonium sulfate solution (40 mL) were added thereto. Then, 6 mol/L hydrochloric acid was added thereto for pH adjustment to 8, and water (80 mL) was added. The organic layer was separated and washed with a 10% aqueous N-acetylcysteine solution (40 mL) once and a mixed solution of a 2.5% aqueous sodium bicarbonate solution and a 20% aqueous sodium chloride solution twice. Anhydrous magnesium sulfate and active carbon were added to the organic layer, and the mixture was stirred. Then, insoluble matter was filtered off. The solvent was distilled off under reduced pressure, and the obtained residue was purified by silica gel column chromatography [eluent: gradient elution of hexane:ethyl acetate = 5:1 → 3:1 → 2:1 → 1:1]. Heptane was added to the obtained purified product, and solid matter was collected by filtration to obtain 12.5 g of 4-(4-((4S)-2,2-dimethyl-1,3-dioxolan-4-yl)phenyl)-2-methyl-3-butyn-2-ol as a white solid.

The obtained solid was used as seed crystals in Example 10.

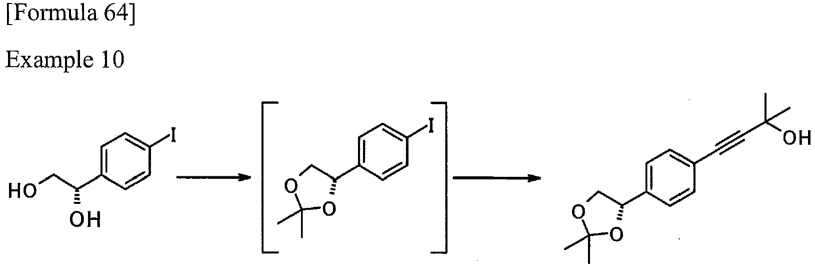

To a mixture of (1S)-1-(4-iodophenyl)ethane-1,2-diol (10 g), N-methylpyrrolidone (30 mL), and 2,2-dimethoxypropane (11.3 mL), methanesulfonic acid (0.25 mL) was added, and the resulting mixture was stirred at room temperature for 9 hours. A solution of triethylamine (11.5 g) in N-methylpyrrolidone (5 mL), bis(triphenylphosphine)palladium(II) dichloride (213 mg), and copper(I) iodide (115 mg) were added to the reaction mixture, then 3-methyl-1-butyn-3-ol (3.82 g) and N-methylpyrrolidone (1 mL) were added thereto at 40°C over 30 minutes in a nitrogen atmosphere, and the mixture was stirred at the same temperature as above for 3.5 hours. The reaction mixture was cooled to room temperature, and ethyl acetate (100 mL) and a 5% aqueous ammonium sulfate solution (50 mL) were added thereto. Then, 6 mol/L hydrochloric acid and a 25% aqueous sodium hydroxide solution were added thereto for pH adjustment to 7.0. The organic layer was separated and washed with a 40% aqueous ammonium sulfate solution (50 mL) once, a 10% aqueous N-acetylcysteine solution (50 mL) once, a 2.5% aqueous sodium bicarbonate solution (50 mL) twice, and a 5% aqueous sodium chloride solution (50 mL) once, and the solvent was distilled off under reduced pressure. The seed crystals and heptane were added to the obtained residue. Solid matter was collected by filtration to obtain 8.1 g of 4-(4-((4S)-2,2-dimethyl-1,3-dioxolan-4-yl)phenyl)-2-methyl-3-butyn-2-ol as a pale yellow solid.
¹H-NMR (400 MHz, CDCl₃) δ:
1.49 (3H, s), 1.55 (3H, s), 1.62 (6H, s), 2.01 (1H, s), 3.67 (1H, td, J = 8.1, 0.6 Hz), 4.30 (1H, dd, J = 8.3, 6.3 Hz), 5.06 (1H, dd, J = 7.6, 6.6 Hz), 7.30 (2H, d, J = 8.5 Hz), 7.41 (2H, d, J = 8.0 Hz).

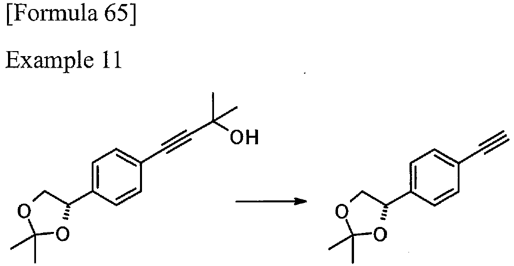

To a solution of 4-(4-((4S)-2,2-dimethyl-1,3-dioxolan-4-yl)phenyl)-2-methyl-3-butyn-2-ol (4.0 g) in toluene (40 mL), potassium tert-butoxide (172 mg) was added, and the solvent (3.4 mL) was distilled off under reduced pressure (300 to 340 Torr) at 78 to 85°C. Water (8 mL) and ethyl acetate (8 mL) were added to the reaction mixture. The organic layer was separated and washed with water (8 mL), and the solvent was distilled off under reduced pressure to obtain 3.56 g of (4S)-4-(4-ethynylphenyl)-2,2-dimethyl-1,3-dioxolane as a brown oil. The obtained brown oil contained toluene at a content of 13%.
¹H-NMR (400 MHz, CDCl₃) δ: 1.49 (3H, s), 1.55 (3H, s), 3.07 (1H, s), 3.67 (1H, t, J = 8.0 Hz), 4.31 (1H, dd, J = 8.3, 6.3 Hz), 5.07 (1H, dd, J = 7.9, 6.5 Hz), 7.32 (2H, d, J = 8.4 Hz), 7.48 (2H, d, J = 8.0 Hz). 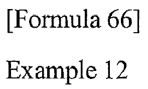 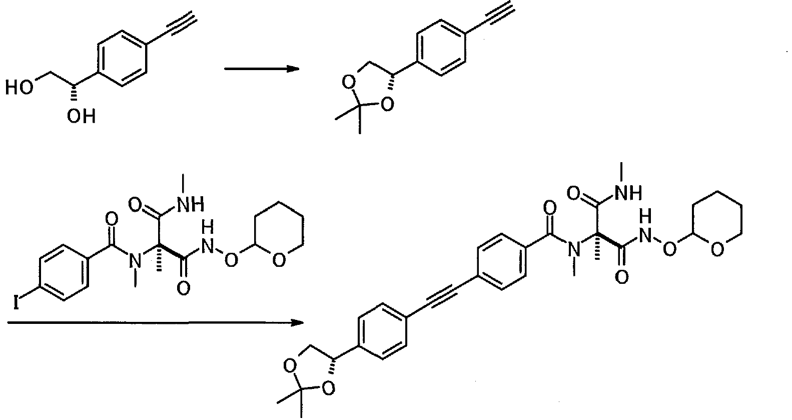
(1) To a mixture of (1S)-1-(4-ethynylphenyl)ethane-1,2-diol (6.96 g) and N-methylpyrrolidone (21 mL), 2,2-dimethoxypropane (13.4 g) and methanesulfonic acid (279 µL) were added in a nitrogen atmosphere, and the resulting mixture was stirred at 20 to 30°C for 5.5 hours to obtain a solution.
(2) A mixture of (2S)-2-((4-iodobenzoyl)(methyl)amino)-N,2-dimethyl-N'-(tetrahydro-2H-pyran-2-yloxy)malo namide (20.0 g), bis(triphenylphosphine)palladium(II) dichloride (0.22 g), copper(I) iodide (0.12 g), triethylamine (10.3 g), and N-methylpyrrolidone (40 mL) was stirred at 30 to 40°C for 1 hour in a nitrogen atmosphere. The solution obtained in (1) was added to the reaction mixture at the same temperature as above over 2.5 hours, and the mixture was stirred at the same temperature as above for 3.5 hours to obtain a reaction mixture.
(3) The same operation as in (1) and (2) was carried out three times. All of the obtained reaction mixtures were combined. 2-Methyltetrahydrofuran (200 mL) and a 5% aqueous ammonium sulfate solution (300 mL) were added to this reaction mixture, and 6 mol/L hydrochloric acid (31 mL) was then added thereto for pH adjustment to 6 to 8. The organic layer was separated.
(4) The same operation as in (1) and (2) was carried out. 2-Methyltetrahydrofuran (200 mL) was added to the obtained reaction mixture, and the mixture was cooled to 0 to 10°C. A 5% aqueous ammonium sulfate solution (100 mL) was added to the obtained mixture, and 6 mol/ hydrochloric acid (17.7 mL) was then added thereto for pH adjustment to 6 to 7. The organic layer was separated.
(5) The same operation as in (4) was carried out.
(6) The organic layers obtained in (3), (4), and (5) were combined and washed with a 40% aqueous ammonium sulfate solution (500 mL) once, a 10% aqueous N-acetylcysteine solution (450 mL) once, a 2.5% aqueous sodium bicarbonate solution (500 mL) twice, and a 5% aqueous sodium chloride solution (500 mL) once, and the solvent was distilled off under reduced pressure. Isopropyl acetate (1200 mL) was added to the obtained residue, and the solvent was distilled off under reduced pressure. Isopropyl acetate was added to the obtained residue, and the mixture was heated to 50°C, then cooled to 25°C over 5 hours, and left standing overnight. Solid matter was collected by filtration and washed with cyclopentyl methyl ether twice to obtain 64.8 g of (2S)-2-((4-((4-((4S)-2,2-dimethyl-1,3-dioxolan-4-yl)phenyl)ethynyl)benzoyl)(methyl)amino)-N,2-dimethyl-N'-(tetrahydro-2H-pyran-2-yloxy)malonamide as a pale yellow solid.

The obtained solid was used as seed crystals in Example 13. 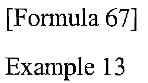 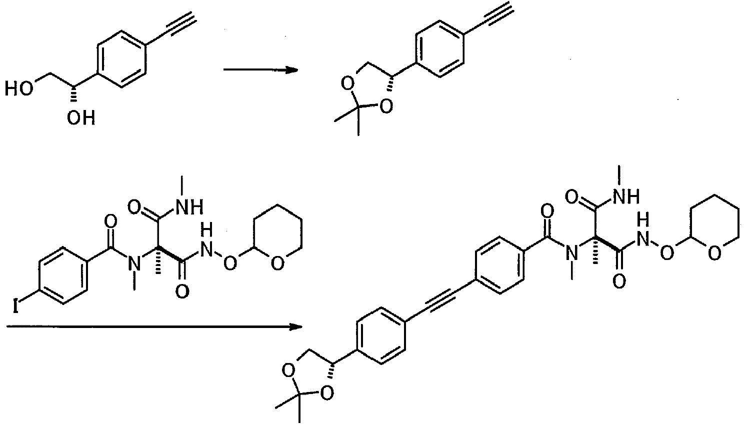
(1) To a mixture of (1S)-1-(4-ethynylphenyl)ethane-1,2-diol (6.96 g) and N-methylpyrrolidone (21 mL), 2,2-dimethoxypropane (13.4 g) and methanesulfonic acid (279 µL) were added in a nitrogen atmosphere, and the resulting mixture was stirred at 20 to 30°C for 5 hours to obtain a solution.
(2) A mixture of (2S)-2-((4-iodobenzoyl)(methyl)amino)-N,2-dimethyl-N'-(tetrahydro-2H-pyran-2-yloxy)malo namide (20.0 g), bis(triphenylphosphine)palladium(II) dichloride (0.22 g), copper(I) iodide (0.12 g), triethylamine (14.5 g), and N-methylpyrrolidone (40 mL) was stirred at 30 to 40°C for 1 hour in a nitrogen atmosphere. The solution obtained in (1) was added to the reaction mixture at the same temperature as above over 3 hours, and the mixture was stirred at the same temperature as above for 2 hours. Ethyl acetate (200 mL) was added to the reaction mixture, and the mixture was cooled to 0 to 10°C. A 5% aqueous ammonium sulfate solution (100 mL) was added thereto, and 6 mol/L hydrochloric acid (14.5 mL) and a 5% aqueous sodium bicarbonate solution (4.5 mL) were then added thereto for pH adjustment to 6 to 7. The organic layer was separated and washed with a 40% aqueous ammonium sulfate solution (100 mL) once, a 10% aqueous N-acetylcysteine solution (100 mL) once, a 5% aqueous sodium bicarbonate solution (100 mL) twice, and water (100 mL) once, and the solvent was distilled off under reduced pressure. Ethyl acetate (160 mL) was added to the obtained residue. After confirmation of dissolution, the solution was heated to 30 to 35°C. Heptane (150 mL) was added to the obtained mixture, then the seed crystals (100 mg) were added thereto, and the resulting mixture was stirred at the same temperature as above for 1 hour. Heptane (50 mL) was added to the obtained mixture, and the resulting mixture was stirred at 30 to 35°C for 1 hour. Heptane (200 mL) was added to the obtained mixture over 1 hour, and the resulting mixture was stirred at the same temperature as above for 1 hour. The obtained mixture was cooled to 20 to 30°C, stirred at the same temperature as above for 30 minutes, and then left standing overnight. The obtained mixture was cooled to 0 to 10°C and stirred at the same temperature as above for 1 hour. Then, solid matter was collected by filtration and washed with a mixed solvent of ethyl acetate and heptane (ethyl acetate:heptane = 1:10) (40 mL) twice to obtain 20.5 g of (2S)-2-((4-((4-((4S)-2,2-dimethyl-1,3-dioxolan-4-yl)phenyl)ethynyl)benzoyl)(methyl)amino)-N,2-dimethyl-N'-(tetrahydro-2H-pyran-2-yloxy)malonamide as a pale yellow solid.
   ¹H-NMR (600 MHz, CDCl₃) δ: 1.50 (3H, s), 1.52-1.74 (3H, m), 1.56 (3H, s), 1.75-1.90 (3H, m), [1.81], 1.82 (3H, s), [2.84], 2.85 (3H, d, J = 4.8 Hz), [3.17], 3.20 (3H, s), [3.53-3.60], 3.62-3.68 (1H, m), 3.70 (1H, t, J = 7.8 Hz), [3.83-3.91], 3.98-4.06 (1H, m), 4.33 (1H, dd, J = 8.4, 6.0 Hz), [4.92-4.98], 4.98-5.03 (1H, m), 5.09 (1H, t, J = 6.0 Hz), [6.98-7.05], 7.61-7.67 (1H, m), 7.37 (2H, d, J = 8.4 Hz), 7.47-7.55 (4H, m), 7.58 (2H, d, J = 8.4 Hz), [10.14], 10.54 (1H, s).

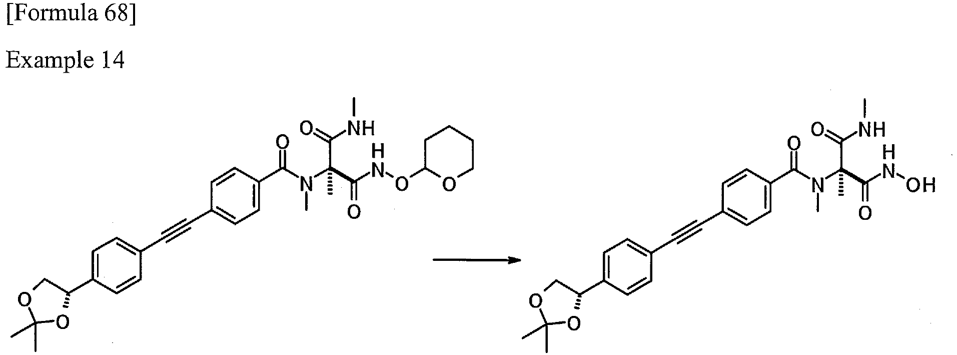

To a mixture of (2S)-2-((4-((4-((4S)-2,2-dimethyl-1,3-dioxolan-4-yl)phenyl)ethynyl)benzoyl)(methyl)amino)-N,2-dimethyl-N'-(tetrahydro-2H-pyran-2-yloxy)malonamide (3.46 g), acetone (3 mL), and 2-propanol (21 mL), methanesulfonic acid (121 µL) was added at 0 to 10°C in a nitrogen atmosphere, and the resulting mixture was stirred at the same temperature as above for 48 hours. 2-Methyltetrahydrofuran (60 mL), a 20% aqueous sodium chloride solution (60 mL), and a 5% aqueous sodium bicarbonate solution (6 mL) were added to the reaction mixture. The organic layer was separated and washed with a 20% aqueous sodium chloride solution (60 mL). Then, active carbon (0.30 g) was added thereto, and the mixture was stirred at 20 to 30°C for 2.5 hours. Insoluble matter was filtered off, and the solvent was distilled off under reduced pressure. Acetonitrile (12 mL) and water (3 mL) were added to the obtained residue, and the mixture was heated to 35 to 40°C. After confirmation of dissolution, water (12.7 mL) was added to the solution, and the mixture was stirred at the same temperature as above for 1 hour. The obtained mixture was cooled to 20 to 30°C, then stirred at the same temperature as above for 1 hour, and left standing overnight. Water (47.9 mL) was added to the obtained mixture over 1 hour, and the resulting mixture was stirred at 20 to 30°C for 1 hour, then cooled to 0 to 10°C, and stirred at the same temperature as above for 3.5 hours. Solid matter was collected by filtration and washed with a 10% aqueous acetonitrile solution (6 mL) twice to obtain 2.65 g of (2S)-2-((4-((4-((4S)-2,2-dimethyl-1,3-dioxolan-4-yl)phenyl)ethynyl)benzoyl)(methyl)amino)-N-hydroxy-N',2-dimethylmalonamide as a white solid.
¹H-NMR (600 MHz, DMSO-d₆) δ: 1.41 (3H, s), 1.47 (3H, s), 1.62 (3H, s), 2.65 (3H, d, J = 4.8 Hz), 3.01 (3H, s), 3.59 (1H, t, J = 8.4 Hz), 4.33 (1H, dd, J = 8.4, 7.2 Hz), 5.11 (1H, t, J = 6.6 Hz), 7.44 (2H, d, J = 8.4 Hz), 7.59 (4H, d, J = 8.4 Hz), 7.65 (2H, d, J = 8.4 Hz), 8.47-8.57 (1H, m), 8.98 (1H, s), 10.97 (1H, s).

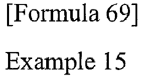

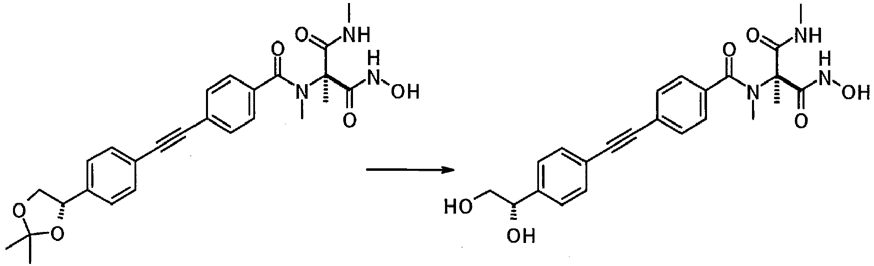

To a mixture of (2S)-2-((4-((4-((4S)-2,2-dimethyl-1,3-dioxolan-4-yl)phenyl)ethynyl)benzoyl)(methyl)amino)-N-hydroxy-N',2-dimethylmalonamide (180 g) and 2-propanol (900 mL), 1 mol/L hydrochloric acid (360 mL) was added at 18 to 20°C, and the resulting mixture was stirred at 20 to 26°C for 10 hours and then left standing overnight. Water (540 mL) was added to the reaction mixture at 24 to 25°C, and the mixture was stirred at 25°C for 45 minutes. Solid matter was collected by filtration and washed with a 50% aqueous 2-propanol solution (180 mL), water (540 mL), and an 80% aqueous 2-propanol solution (540 mL) in this order to obtain 114 g of (2S)-2-((4-((4-((1S)-1,2-dihydroxyethyl)phenyl)ethynyl)benzoyl)(methyl)amino)-N-hydroxy-N',2-dimethylmalonamide hydrate as a white solid.

The obtained solid was used as seed crystals in Example 16.

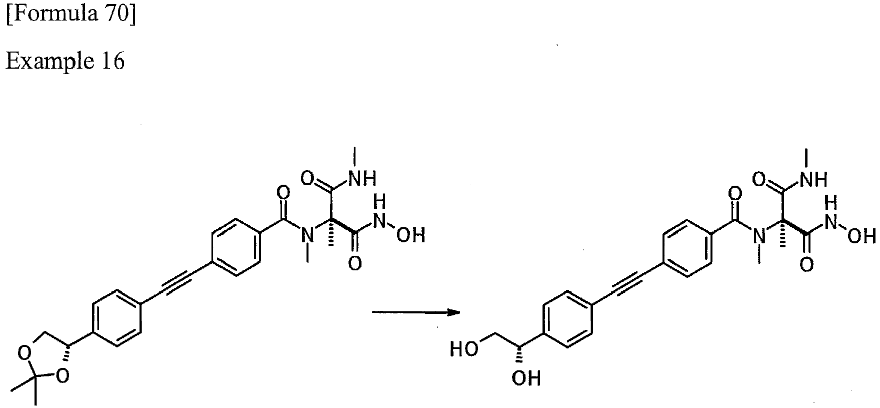

To a mixture of (2S)-2-((4-((4-((4S)-2,2-dimethyl-1,3-dioxolan-4-yl)phenyl)ethynyl)benzoyl)(methyl)amino)-N-hydroxy-N',2-dimethylmalonamide (2.00 g) and acetonitrile (20 mL), 1 mol/L hydrochloric acid (4 mL) was added at 5 to 10°C, and the resulting mixture was stirred at the same temperature as above for 18.5 hours. A mixture of sodium bicarbonate (0.34 g) and water (8.1 mL) was added to the reaction mixture, and the resulting mixture was heated to 50 to 60°C. After confirmation of dissolution, the pH of the solution was adjusted to 5 to 6 using a 5% aqueous sodium bicarbonate solution and 1 mol/L hydrochloric acid. The obtained mixture was cooled to 35 to 40°C. The seed crystals (10 mg) were added thereto, and the mixture was stirred at the same temperature as above for 1 hour. The obtained mixture was cooled to 30 to 35°C. Water (47 mL) was added thereto over 2 hours, and the mixture was stirred at the same temperature as above for 1 hour. The obtained mixture was cooled to 22.5 to 27.5°C, stirred at the same temperature as above for 1 hour, and then left standing overnight. The obtained mixture was cooled to 12.5 to 17.5°C and stirred at the same temperature as above for 1 hour. The obtained mixture was cooled to 0 to 5°C and then stirred at the same temperature as above for 3 hours. Solid matter was collected by filtration and washed with a 10% aqueous acetonitrile solution (2 mL) twice to obtain 1.62 g of (2S)-2-((4-((4-((1S)-1,2-dihydroxyethyl)phenyl)ethynyl)benzoyl)(methyl)amino)-N-hydroxy-N',2-dimethylmalonamide hydrate as a white solid. This solid was defined as type II crystal.

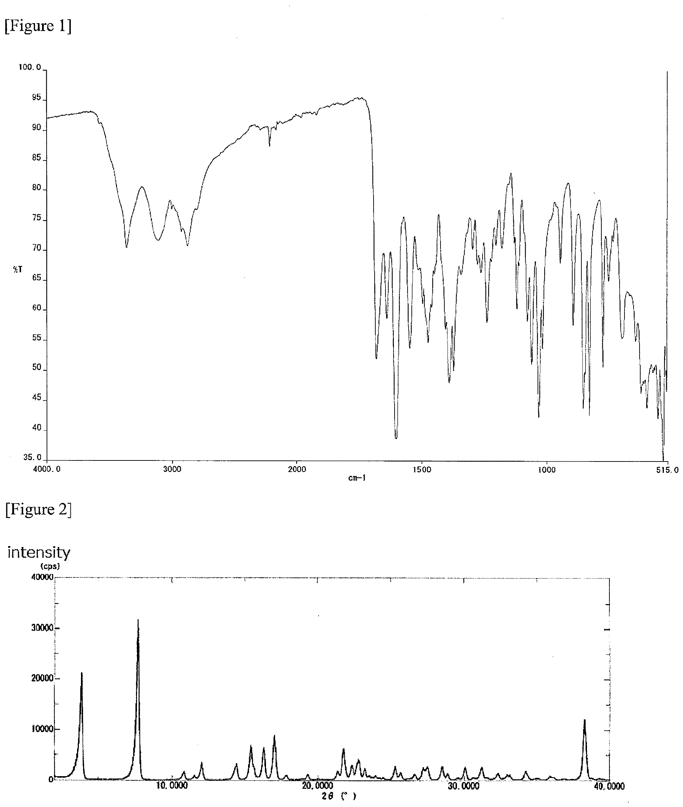

The attenuated total reflection infrared spectroscopy (ATR method) is shown in Figure 1.

The powder X-ray diffraction pattern is shown in Figure 2 and Table 1.
Water content: 4.1%
IR (ATR): 1474, 1605, 1682, 3132, 3363cm⁻¹
Optical purity: >99%ee (>99%de)

### HPLC measurement conditions

Measurement wavelength : 290 nm
Column: CHIRALCEL OZ-H (Daicel Corporation), particle diameter: 5 µm, inner diameter 4.6 mm × length 250 mm
Column temperature: 40°C
Flow rate: 0.7 mL/min
Mobile phase: hexane/ethanol/acetic acid = 650/350/5
¹H-NMR (600MHz, DMSO-d₆) δ value: 1.62 (3H, s), 2.65 (3H, d, J = 3.6Hz), 3.01 (3H, s), 3.40-3.51 (2H, m), 4.53-4.62 (1H, m), 4.77 (1H, t, J = 5.4Hz), 5.36 (1H, d, J = 4.2Hz), 7.41 (2H, d, J = 7.8Hz), 7.53 (2H, d, J = 7.8Hz), 7.58 (2H, d, J = 7.8Hz), 7.64 (2H, d, J = 8.4Hz), 8.52 (1H, d, J = 4.2Hz), 8.98 (1H, s), 10.97 (1H, s).

**[Table 1]**

| 2θ (°) | d value (Å) | Relative intensity (%) |
|---|---|---|
| 3.8 | 23.1 | 74 |
| 7.7 | 11.5 | 100 |
| 10.8 | 8.2 | 6 |
| 12.0 | 7.3 | 12 |
| 14.4 | 6.1 | 11 |
| 15.4 | 5.7 | 25 |
| 16.3 | 5.4 | 24 |
| 17.0 | 5.2 | 33 |
| 21.8 | 4.1 | 23 |
| 22.3 | 4.0 | 10 |
| 22.8 | 3.9 | 13 |

### Example 17

49.5 mL of a methanol suspension containing 16.5 g of type II crystal was stirred at a temperature of 20°C to 30°C for 3 hours. Thereafter, a solid was collected by filtration, and was then washed with 33 mL of methanol twice to obtain 12.0 of a white solid. This solid was defined as type III crystal.

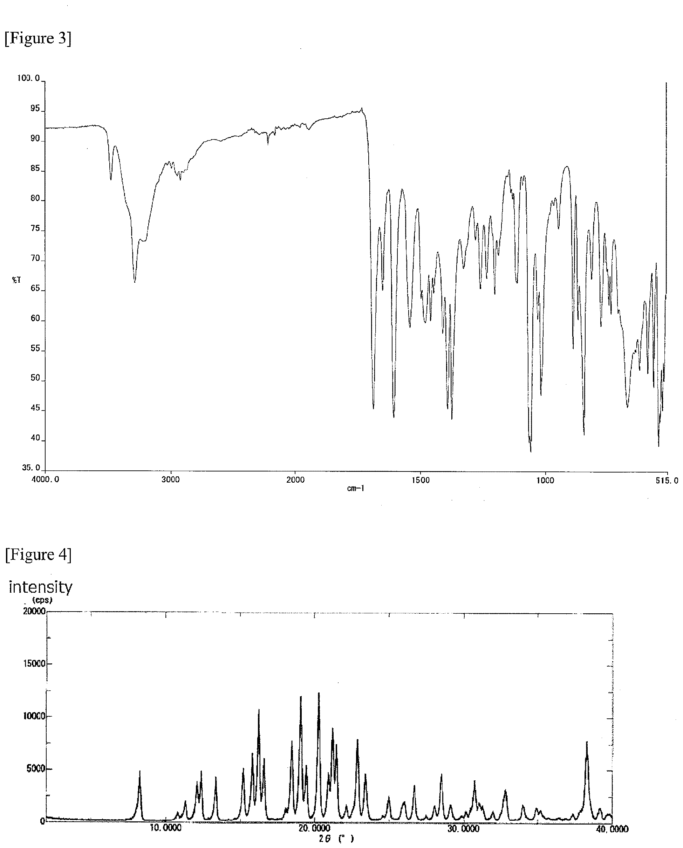

The attenuated total reflection infrared spectroscopy (ATR method) is shown in Figure 3.

The powder X-ray diffraction pattern is shown in Figure 4 and Table 2.

The obtained solid was used as a seed crystal in Example 18.
Water content: 0.3%
IR (ATR): 1481, 1607, 1688, 3286, 3475 cm⁻¹
¹H-NMR (400MHz, DMSO-d₆) δ value: 1.63 (3H, s), 2.65 (3H, d, J = 4.8Hz), 3.02 (3H, s), 3.40-3.51 (2H, m), 4.53-4.62 (1H, m), 4.77 (1H, t, J = 5.8Hz), 5.37 (1H, d, J = 4.4Hz), 7.41 (2H, d, J = 8.0Hz), 7.53 (2H, d, J = 8.4Hz), 7.59 (2H, d, J = 8.0Hz), 7.64 (2H, d, J = 8.4Hz), 8.46-8.58 (1H, m), 8.99 (1H, d, J = 1.2Hz), 10.98 (1H, s).

**[Table 2]**

| 2θ (°) | d value (Å) | Relative intensity (%) |
|---|---|---|
| 8.3 | 10.7 | 33 |
| 12.4 | 7.1 | 40 |
| 13.4 | 6.6 | 31 |
| 15.2 | 5.8 | 40 |
| 15.8 | 5.6 | 52 |
| 16.2 | 5.5 | 90 |
| 16.6 | 5.3 | 47 |
| 18.5 | 4.8 | 64 |
| 19.1 | 4.7 | 100 |
| 19.4 | 4.6 | 42 |
| 20.3 | 4.4 | 100 |
| 20.9 | 4.2 | 38 |
| 21.2 | 4.2 | 77 |
| 21.5 | 4.1 | 60 |
| 22.9 | 3.9 | 67 |
| 23.4 | 3.8 | 39 |

### Example 18

To a mixed solution of 2 mL of methanol and 0.5 mL of dimethyl sulfoxide, 1.00 g of type II crystal was added at a temperature of 0°C to 10°C under a nitrogen atmosphere, and the obtained mixture was then stirred at the same temperature as mentioned above for 4 minutes. After confirmation of the dissolution of the crystal, the temperature was increased to a temperature of 20°C to 25°C, and 2 mL of methanol and 2 mL of ethyl acetate were then added to the reaction mixture. Thereafter, a seed crystal of the type III crystal was added to the mixture, and the thus obtained mixture was then stirred at the same temperature as mentioned above for 2 hours. Thereafter, 8 mL of ethyl acetate was added to the reaction mixture at a temperature of 20°C to 25°C over 30 minutes, and was then stirred at the same temperature as mentioned above for 1 hour. Subsequently, 10 mL of heptane was added to the reaction mixture at a temperature of 20°C to 25°C over 30 minutes, and was then stirred at the same temperature as mentioned above for 1 hour. After that, the reaction mixture was cooled to a temperature of 0°C to 10°C, and was then stirred at the same temperature as mentioned above for 1 hour. A solid was collected by filtration, and was then washed with 2 mL of ethyl acetate twice to obtain 0.90 g of type III crystal in the form of a white solid.

The attenuated total reflection infrared spectroscopy (ATR method) and the powder X-ray diffraction pattern were matched with those of Example 17.
Water content: 0.1%
Optical purity: >99%ee (>99%de)

### HPLC measurement conditions

Measurement wavelength : 290 nm
Column: CHIRALCEL OZ-H (Daicel Corporation), particle diameter: 5 µm, inner diameter 4.6 mm × length 250 mm
Column temperature: 40°C
Flow rate: 0.7 mL/min
Mobile phase: hexane/ethanol/acetic acid = 650/350/5

### Industrial Applicability

The production method of the present invention is useful as an industrial method for producing a compound having excellent LpxC inhibitory activity. The compound of the present invention is useful as an intermediate for use in the industrial production.

## Claims

1. A method for producing a compound represented by formula [2], comprising :
deprotecting a compound represented by the formula [1]: 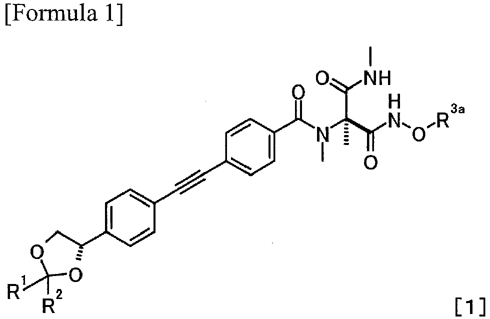
wherein R¹ represents a C₁₋₃ alkyl group; R² represents a C₁₋₃ alkyl group; and R^{3a} represents a hydroxyl-protecting group
to produce the compound represented by formula [2]:

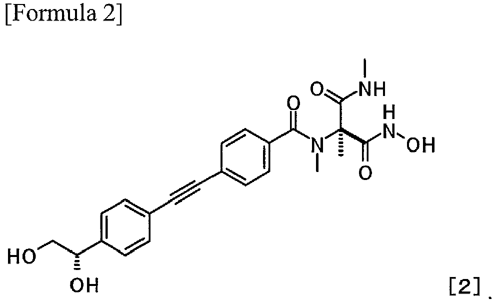

2. The production method according to claim 1, wherein the deprotecting of the compound represented by the formula [1] comprises:
(1) deprotecting the hydroxyl-protecting group in the compound represented by the formula [1] to obtain a compound represented by the formula [3]: 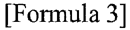 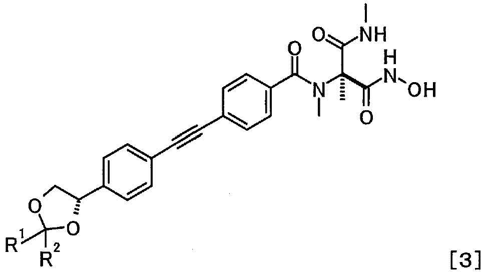 wherein R¹ represents a C₁₋₃ alkyl group; and R² represents a C₁₋₃ alkyl group; and
(2) deprotecting the compound represented by the formula [3] to obtain the compound represented by formula [2]:

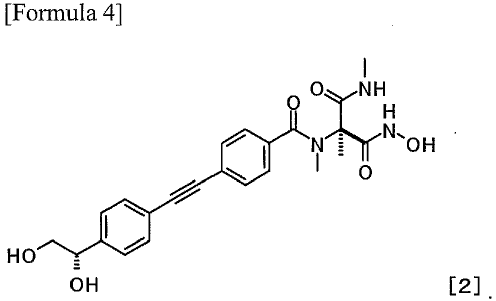

3. The production method according to claim 1 or 2, wherein R¹ is a methyl group; and R² is a methyl group.

4. The production method according to any one of claims 1 to 3, wherein R^{3a} is a tetrahydropyranyl group.

5. A method for producing a compound represented by formula [2], comprising:
reacting a compound represented by the formula [4]: 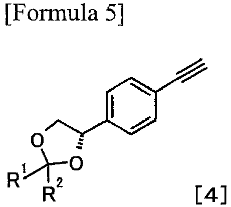
wherein R¹ represents a C₁₋₃ alkyl group; and R² represents a C₁₋₃ alkyl group
with a compound represented by the formula [5]: 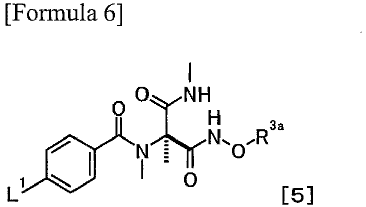
wherein R^{3a} represents a hydroxyl-protecting group; and L¹ represents a leaving group to obtain a compound represented by the formula [1]: 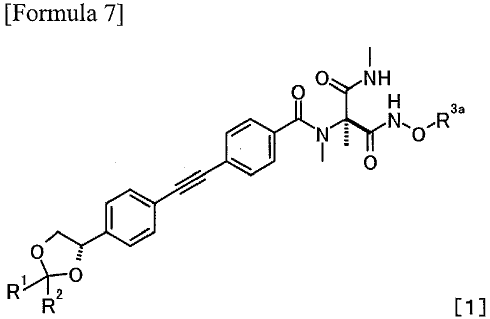
wherein R¹, R², and R^{3a} are as defined above; and
deprotecting the obtained compound represented by the formula [1] to produce the compound represented by formula [2]:

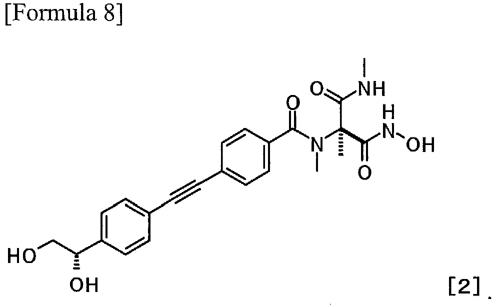

6. The production method according to claim 5, wherein the deprotecting of the compound represented by the formula [1] comprises:
(1) deprotecting the hydroxyl-protecting group in the compound represented by the formula [1] to obtain a compound represented by the formula [3]: 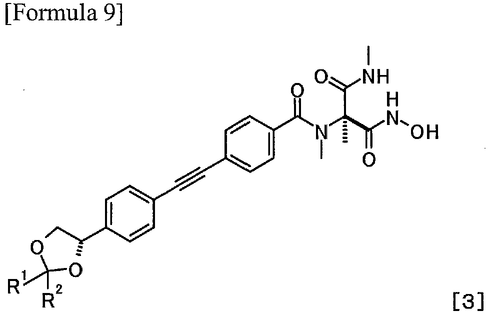 wherein R¹ represents a C₁₋₃ alkyl group; and R² represents a C₁₋₃ alkyl group; and
(2) deprotecting the compound represented by the formula [3] to obtain the compound represented by formula [2]:

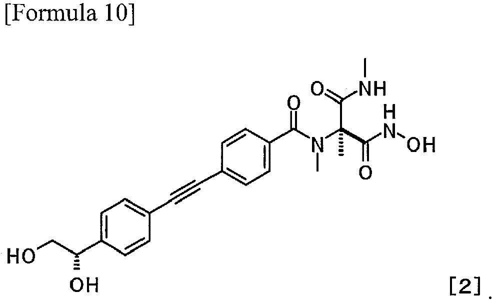

7. The production method according to claim 5 or 6, wherein R¹ is a methyl group; and R² is a methyl group.

8. The production method according to any one of claims 5 to 7, wherein R^{3a} is a tetrahydropyranyl group; and L¹ is an iodine atom.

9. A method for producing a compound represented by formula [2], comprising:
reducing a compound represented by the formula [6]: 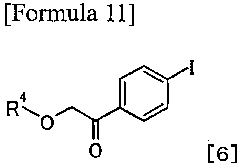
wherein R⁴ represents an optionally substituted acyl group
to obtain a compound represented by the formula [7]: 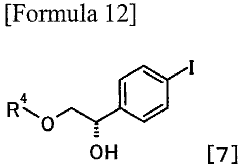
wherein R⁴ is as defined above;
deprotecting the obtained compound represented by the formula [7] to obtain a compound represented by formula [8]: 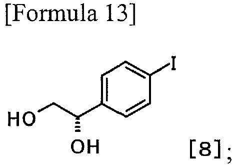
reacting the obtained compound represented by formula [8] with a compound represented by the formula [9]: 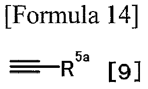
wherein R^{5a} represents a silyl group
to obtain a compound represented by the formula [10]: 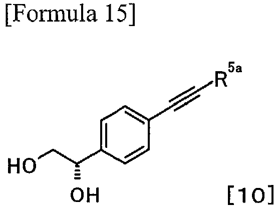
wherein R^{5a} is as defined above;
deprotecting the obtained compound represented by the formula [10] to obtain a compound represented by formula [11]: 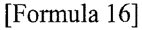 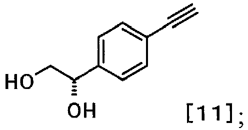
protecting the diol group in the obtained compound represented by formula [11] to obtain a compound represented by the formula [4]: 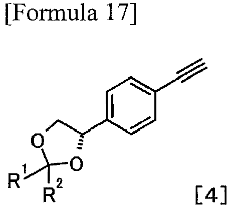
wherein R¹ represents a C₁₋₃ alkyl group; and R² represents a C₁₋₃ alkyl group;
reacting the obtained compound represented by the formula [4] with a compound represented by the formula [5]: 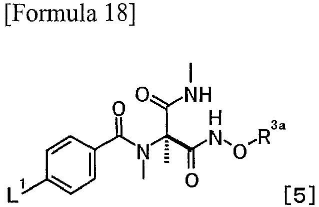
wherein R^{3a} represents a hydroxyl-protecting group; and L¹ represents a leaving group to obtain a compound represented by the formula [1]: 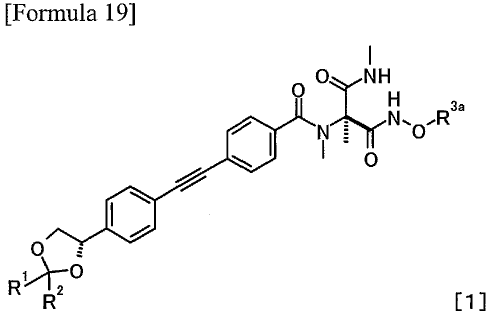
wherein R¹, R², and R^{3a} are as defined above; and
deprotecting the obtained compound represented by the formula [1] to produce the compound represented by formula [2]:

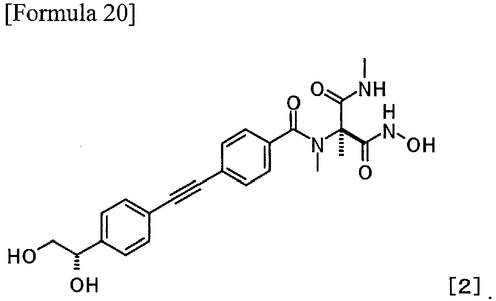

10. The production method according to claim 9, wherein the deprotecting of the compound represented by the formula [1] comprises:
(1) deprotecting the hydroxyl-protecting group in the compound represented by the formula [1] to obtain a compound represented by the formula [3]: 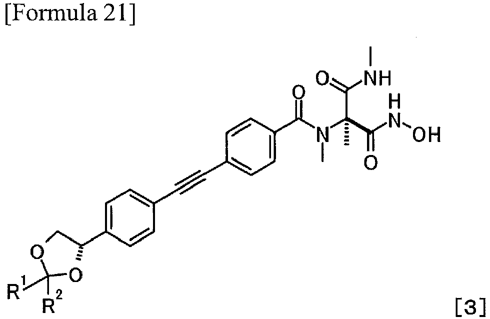 wherein R¹ represents a C₁₋₃ alkyl group; and R² represents a C₁₋₃ alkyl group; and
(2) deprotecting the compound represented by the formula [3] to obtain the compound represented by formula [2]:

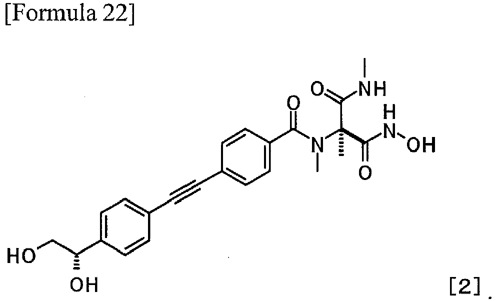

11. The production method according to claim 9 or 10, wherein R⁴ is an acetyl group; and R^{5a} is a trimethylsilyl group.

12. The production method according to any one of claims 9 to 11, wherein R¹ is a methyl group; and R² is a methyl group.

13. The production method according to any one of claims 9 to 12, wherein R^{3a} is a tetrahydropyranyl group; and L¹ is an iodine atom.

14. A method for producing a compound represented by formula [8], comprising:
reducing a compound represented by the formula [6]: 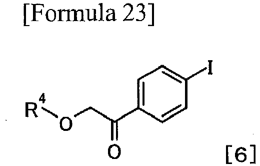
wherein R⁴ represents an optionally substituted acyl group
to obtain a compound represented by the formula [7]: 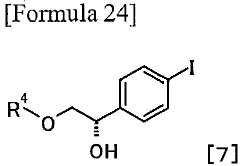
wherein R⁴ is as defined above; and
deprotecting the obtained compound represented by the formula [7] to produce the compound represented by formula [8]:

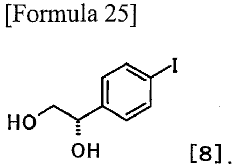

15. The production method according to claim 14, wherein R⁴ is an acetyl group.

16. A compound represented by the formula [12]: 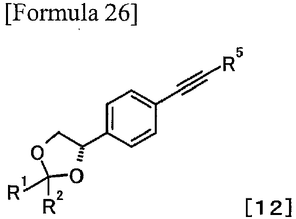 wherein R¹ represents a C₁₋₃ alkyl group; R² represents a C₁₋₃ alkyl group; and R⁵ represents a hydrogen atom, a group represented by the formula [13]: 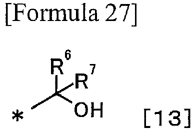 wherein R⁶ represents a C₁₋₃ alkyl group; R⁷ represents a C₁₋₃ alkyl group; or R⁶ and R⁷ together represent a C₃₋₆ alkylene group; and * represents a binding position, or
a group represented by the formula [14]: 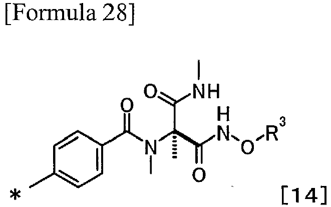 wherein R³ represents a hydrogen atom or a hydroxyl-protecting group; and * represents a binding position.

17. The compound according to claim 16, wherein R¹ is a methyl group; R² is a methyl group; and R⁵ is a hydrogen atom.

18. The compound according to claim 16, wherein R¹ is a methyl group; R² is a methyl group; and R⁵ is a group represented by the formula [14]: 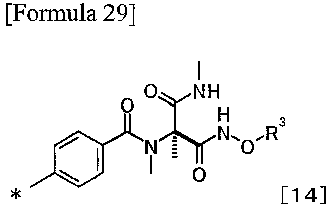 wherein R³ represents a hydrogen atom or a hydroxyl-protecting group; and * represents a binding position.

19. The compound according to claim 18, wherein R³ is a hydrogen atom or a tetrahydropyranyl group.
